# EUROPÄISCHE PATENTSCHRIFT

(11) **EP 1 023 453 B1**
(45) Veröffentlichungstag und Bekanntmachung des Hinweises auf die Patenterteilung: **20.12.2006**
(21) Anmeldenummer: 98966300.0
(22) Anmeldetag: 11.12.1998
(51) Int. Cl.: C12N 15/87, C12N 15/85, C07K 14/195, C12N 1/21, C12R 1/01, A01K 67/027, A61K 48/00

(54) **TGC-VERFAHREN ZUR INDUKTION EINER ZIELGERICHTETEN, SOMATISCHEN TRANSGENITÄT**
TGC METHOD FOR INDUCTING TARGETED SOMATIC TRANSGENESIS
PROCEDE DE CONDITIONNEMENT GENETIQUE CIBLE POUR L'INDUCTION D'UNE TRANSGENESE CIBLEE SOMATIQUE

(30) Priorität: 11.12.1997 DE 19754938
(43) Veröffentlichungstag der Anmeldung: 02.08.2000
(73) Patentinhaber: Von Eichel-Streiber, Christoph, 55444 Schweppenhausen (DE); Chakraborty, Trinad, 35390 Giessen (DE)
(72) Erfinder: Von Eichel-Streiber, Christoph, 55444 Schweppenhausen (DE); Chakraborty, Trinad, 35390 Giessen (DE)
(74) Vertreter: Rudolph, Ulrike
(86) Internationale Anmeldenummer: PCT/EP1998/008096
(87) Internationale Veröffentlichungsnummer: WO 1999/029884

(56) Entgegenhaltungen:
- WO-A-93/15212
- WO-A-96/40238
- WO-A-97/08955
- WO-A-98/44131
- WO-A-98/48026
- FR-A- 2 743 086
- COURVALIN P ET AL: "GENE TRANSFER FROM BACTERIA TO MAMMALIAN CELLS" COMPTES RENDUS DES SEANCES DE L'ACADEMIE DES SCIENCES SERIE III: SCIENCES DE LA VIE, Bd. 318, Nr. 12, 1. Dezember 1995, Seiten 1207-1212, XP000579701
- PASCUAL D W ET AL: "ORAL BACTERIAL VACCINE VECTORS FOR THE DELIVERY OF SUBUNIT AND NUCLEIC ACID VACCINES TO THE ORGANIZED LYMPHOID TISSUE OF THE INTESTINE" BEHRING INSTITUTE: MITTEILUNGEN, Bd. 98, Februar 1997, Seiten 143-152, XP002910650
- T. CHAKRABORTY AND J. WEHLAND: "The host cell infected with Listeria monocytogenes in" HOST RESPONSE TO INTRACELLULAR PATHOGENS, ED. S.H.E. KAUFMANN R.G. LANDES CO.,1997, Seiten 271-290, XP002104783 Austin, US in der Anmeldung erwähnt
- S. DRAMSI ET AL.: "Identification of four new members of the internalin multigen family of Listeria monocytogenes EGD" INFECTION AND IMMUNITY, Bd. 65, Nr. 5, Mai 1997, Seiten 1615-1625, XP002104784 ASM,WASHINGTON,DC,US
- G. DIETRICH ET AL.: "Delivery of antigen-encoding plasmid DNA into the cytosol of macrophages by attenuated suicide Listeria monocytogenes" NATURE BIOTECHNOLOGY, Bd. 16, Nr. 1, Januar 1998, Seiten 181-185, XP002104785 NATURE PUBL. CO.,NEW YORK, US
- C.A. GUZMÁN ET AL.: "Attenuated Listeria monocytogenes carrier strains can deliver an HIV-1 gp120 T helper epitope to MHC class II-restricted guman CD4+ T cells" EUROPEAN J. IMMUNOLOGY, Bd. 28, Juni 1998, Seiten 1807-1814, XP002104786 VCH VERLAGSGESELLSCHAFT MBH, WEINHEIM, BRD
- C. GRILLOT-COURVALIN ET AL.: "Functional gene transfer from intracellular bacteria to mammalian cells" NATURE BIOTECHNOLOGY, Bd. 16, Nr. 9, September 1998, Seiten 862-866, XP002104787 NATURE PUBL. CO.,NEW YORK, US
- D. RAFFELSBAUER ET AL.: "The gene cluster inlC2DE of Listeria monocytogenes contains additional new internalin genes and is important for virulence in mice" MOLECULAR & GENERAL GENETICS, Bd. 260, Nr. 2,3, November 1998, Seiten 144-158, XP002104788 SPRINGER INTERNATIONAL, AMSTERDAM, NL

## Beschreibung

Gegenstand der Erfindung sind Bakterien zur Verwendung als Vehikel zum Gentransport und Gentransfer in eukaryonten Zellen eines Organismus zur Induktion einer zielgerichteten somatischen Transgenität (TGC = targeted genetic conditioning) in Zellen, Geweben oder Organen, ausgenommen der Keimbahn des Organismus, wobei die Bakterien eine in einen episomalen Vektor integrierte Fremd-DNA aufweisen, deren Transkription und Expression unter der Kontrolle eukaryonter, regulatorischer Elemente steht, sowie ein Verfahren zur Gewinnung von Proteinen, bei dem solche Bakterien eingesetzt werden und in den infizierten und transgenen Zellen die Bildung des Fremd-Proteins initiieren, das anschließend aus der Zelle oder dem Gewebe oder dem Organ mit fachüblichen Methoden isoliert und gereinigt wird.

Es ist bekannt, daß Proteine für technische Anwendungen oder für therapeutische Zwecke durch den Transfer von Genen in Mikroorganismen oder Säugetierzellen in ausreichenden Mengen exprimiert werden können. Diese Verfahren sind besonders bedeutsam für körpereigene Proteine, die sonst nicht oder nur begrenzt zugänglich sind, wie Hormone, Regulationsfaktoren, Enzyme, Enzyminhibitoren und humanisierte monoklonale Antikörper sowie für die Herstellung von Oberflächenproteinen pathogener Mikroorganismen oder viraler Hüllproteine zur gefahrlosen Herstellung diagnostischer Tests und verträglicher Impfstoffe. Durch "Protein-Engineering" können auch neuartige Proteine hergestellt werden, die durch Fusion, Mutation oder Deletion entsprechender DNA-Sequenzen anwendungs-optimierte Eigenschaften erhalten, zum Beispiel Immuntoxine.

Aus menschlichen Zellen gewonnene Gene sind auch in Maus-, Ratten- oder Schafzellen funktionsfähig und führen dort zur Bildung entsprechender Genprodukte. Dies wurde bereits praktisch bei der Herstellung von therapeutischen Proteinen, zum Beispiel in der Milch transgener Nutztiere, angewendet. Der bisher bekannte Weg hierfür ist die Mikroinjektion entsprechender Fremd-DNA-tragender Vektoren in den Kern der befruchteten Eizelle, in der bei einer Ausbeute von 1% die DNA dann in das Chromossom eingebaut wird. Die transgene befruchtete Eizelle wird anschließend hormonell stimulierten Muttertieren reimplantiert. Ein Nachkomme, der das eingeschleuste Gen in allen Körperzellen trägt, ist die Grundlage für die Bildung einer "transgenen Herde". Durch die Nutzung der Gentechnik ist es möglich geworden, landwirtschaftliche Nutztiere so gezielt zu verändern, daß sie menschliche Proteine in ihrem Blut, ihrem Gewebe oder der Milch produzieren, die in Mikroorganismen oder Pflanzen nicht hergestellt werden können.

Der Einsatz von transgenen Tieren als Proteinproduktionsfabriken hat jedoch den entscheidenden Nachteil, daß hierzu ein Eingriff in die Keimbahn der Tiere erforderlich ist. Wegen des hohen technischen und zeitlichen Aufwandes zur Schaffung und Züchtung von transgenen Tieren und auch wegen der Diskussion über die ethischen Konsequenzen dieser Methoden sind alternative Methoden zur Proteinproduktion in einem animalen Wirt ohne Keimbahneingriff erforderlich und wären von sehr großem Vorteil.

Aus der FR A 2 743 086 ist die Anwendung von genetisch modifizierten, nämlich mit pWR100 von Shigella flexneri ausgerüsteten, apathogenen, nicht kolonisierungsfähigen E.coli K12 Bakterien als Vehikel zum Gentransport und Gentransfer in eukaryontische Zellen bekannt. Hierbei müssen die E.coli K12 Bakterien mit den zu transfizierenden eukaryonten Zellen zunächst in direkten räumlichen Kontakt gebracht werden, d.h. sie müssen direkt zu bzw. auf die betreffenden eukaryonten Zellen appliziert werden. Die derart applizierten Bakterien können dann in das Zytoplasma der eukaryonten Zelle eindringen, können dort aber aufgrund einer DAP-Auxotrophie nicht überleben, sondern sterben ab und setzten dabei ihre DNA einschließlich der Fremd-DNA frei.

Damit ist die betreffende eukaryonte Zelle transgen geworden. Zellen, die abseits des Applikationsorts liegen, z.B. in tieferen Gewebeschichten, können von diesen Bakterien nicht erreicht und infolgedessen nicht transfiziert werden.

Courvalin P et al. (Bd 318, Nr 12, 1.12.1995 S. 1207-1212, XP000579701) beschreiben ähnliche Untersuchungsergebnisse für Shigella flexneri selbst. Shigella flexneri ist zwar pathogen, aber nur für Epithelzellen. Die Shigellen können die Schicht der Epithelzellen nicht überwinden, sondern bleiben auf den Applikationsort, nämlich auf die erstinfizierten Epithelzellen (sogn. Eintrittspforten) beschränkt. Die Freisetzung der DNA ins Zytoplasma der Zielzelle (ausschließlich Epithelzellen) erfolgt wiederum nach Eintritt der Bakterien ins Zytoplasma der Zelle und nachfolgender Lyse der Bakterien vor Ort. Eintrittspforte und Zielzelle (für Infektion und Transfektion) sind identisch.

In der WO 97 08955 ist der Einsatz von attenuierten (in ihrer Virulenz abgeschwächten) Bakterien zum Gentransport in eukaryonte Zellen beschrieben. Die attenuierten Bakterien sind so konzipiert, daß sie keine Krankheit induzieren, aber dennoch ins Zytoplasma von Zellen eindringen können. Im Zytoplasma der infizierten Zelle sterben die Bakterien, wobei sie ihre DNA in diese Zelle freigeben. Die attenuierten Bakterien müssen mit den zu transfizierenden eukaryonten Zellen zunächst in direkten räumlichen Kontakt gebracht werden, um eindringen, absterben und die DNA freisetzen zu können. Der DNA-Transfer erfolgt somit ausschließlich in die primär infizierten Zellen des Applikationsorts.

Aus der WO 96 40238 A ist die heterologe Expression von sogenannten Suizidgenen in Bakterien beschrieben. Diese "Suizidgene" sind jedoch streng genommen "Killergene", denn sie werden zur Erzeugung von heterolog exprimierten "Killerproteinen" eingesetzt. Die "Killerproteine" werden von den Bakterien in eukaryonte Zielzelle eingeschleust und führen dort den Tod der infizierten eukaryonten Zelle (vorzugsweise Tumorzelle) - nicht der Bakterienzelle - herbei.

In der WO 97/08955 (Brandstrom et al.) ist der Einsatz von attenuierten Shigellen zum Gentransport in eukaryonte Zellen beschrieben. Die attenuierten Shigellen sind so konzipiert, daß sie keine Krankheit induzieren, aber dennoch ins Zytoplasma ihrer Zielzellen, nämlich der Zellen der Magen-Darm-Schleimhaut, eindringen können. Im Zytoplasma der infizierten Zelle sterben die Bakterien, wobei sie ihre DNA in diese Zelle freigeben. Da Shigellen nur in denjenigen Zellen eine Infektion erzeugen, in die sie direkt eingedrungen sind, beinhaltet die Lehre der D5, daß der gewünschte DNA-Transfer ausschließlich in die primär infizierten Zellen des Applikationsortes, nämlich in die Enterozyten der Magen-Darm-Schleimhaut erfolgt. Die applizierten Shigellen sind nicht in der Lage, den Wirt zu kolonisieren und eine Infektion und damit einen DNA-Transfer abseits des primären Applikationsorts zu erzeugen.

Es ist weiterhin bekannt, daß die Milch von Säugetieren wie Kühen, Schafen, Ziegen, Pferden oder Schweinen eine Reihe von bakteriellen Krankheitserregern enthalten kann. Darunter sind Listerien, Mykobakterien, Brucellen, Rhodococcus, Salmonellen, Shigellen, Escherichia, Aeromonaden und Yersinien, oder generell Bakterien mit intrazellulärem Lebensstil [1, 2]. Diese Bakterien werden im wesentlichen durch orale Aufnahme auf den Menschen oder das Tier übertragen [3], aber auch Tröpfcheninfektionen spielen eine Rolle. Eine Hauptquelle für die Infektion des Menschen mit Listerien [4], Mykobakterien [5] und Escherichia coli ist kontaminierte Milch [6]. Der Mensch nimmt die Bakterien beim Verzehr nicht pasteurisierter Milch oder Milchprodukte auf. Die anderen oben aufgeführten Bakteriengattungen wie Salmonellen, Shigellen, Yersinien, Rhodococcus und Brucellen werden in ähnlicher Weise auf den Menschen übertragen. Bakterien können aber auch durch andere mit Bakterien infizierte Tierprodukte aus der Kuh, der Ziege, dem Schaf, dem Hasen, dem Pferd, dem Schwein oder dem Geflügel in den Menschen gelangen.

Die Infektion der Tiere erfolgt dabei oft über mukosale Oberflächen, sehr häufig über den Verdauungstrakt. Nach der Aufnahme von Bakterien werden zum Beispiel im Fall von Listerien jedoch nicht alle Gewebe symptomatisch infiziert. Bei der Kuh und bei der Ziege steht die Infektion von Euter, Milz und Leber im Vordergrund des Infektionsgeschehens. Bei Schafen kann es außerdem auch zur Erkrankung des zentralen Nervensystems in Form der Meningitis kommen, so daß nicht alle Tiere die Infektion überleben. Mit der Infektion des Euters ist die Infektionskette geschlossen. Mit kontaminierter Milch aufgenommene Bakterien können dann das Tier, zum Beispiel das saugende Kalb, oder den Menschen über den Verdauungstrakt neu infizieren.

Über den Weg der bakteriellen Infektion des Menschen, hier speziell dargestellt am Beispiel der Listerien, ist derzeit folgendes bekannt:

Für den Menschen sind von den sechs bekannten Listeria-Spezies lediglich L.monocytogenes und L.ivanovii [7] pathogen. Die Erkrankung des Menschen erfolgt beim Verzehr infizierter Milch oder Milchprodukte. Der Verlauf der Infektion hängt vom Gesundheitszustand des Menschen ab und ist in aller Regel undramatisch. In der Schwangerschaft kann es zu der intrauterinen Übertragung des Keims auf den Fetus kommen, verbunden mit Fehl-, Tod- oder Frühgeburten. In allen Fällen besteht eine exzellente und problemlose Behandelbarkeit mit Antibiotika wie Ampicillin oder Erythromycin [8; 8a].

Für L.monocytogenes in Mensch und Tier, für L.ivanovii im Schaf, ist der Weg in die Zelle gut definiert. Zur vollen Pathogenität der Listerien sind eine Reihe von Pathogenitätsfaktoren notwendig. Dazu gehört PrfA (positive regulator of virulence), ActA (actin nucleating protein), PlcA (phosphatidylinositol-specific phospholipase), PlcB (phosphatidylcholine-specific phospholipase), Hly (listeriolysin), Mpl (metalloprotease) [9]. Die Zellspezifität der Pathogen-Wirtszelle-Interaktion wird über eine Reihe von Proteinen vermittelt. Dazu gehören die Internaline InlA und InlB, die am initialen Kontakt und der Interaktion von Bakterien und Zelloberfläche beteiligt sind [10, 11]. L.monocytogenes kann in der experimentellen Situation unter anderem Endothelzellen, Epithelzellen, Fibroblasten und Hepatozyten infizieren. Darüber hinaus infiziert L.monocytogenes mit neutrophilen Granulocyten, Makrophagen und Lymphozyten auch Zellen des weißen Blutbildes. Dies ist ein wesentlicher Faktor bei der Übertragung der Bakterien von der Eintrittspforte zum Zielorgan im Wirt. Schließlich kann auch das Lungengewebe durch Listerien infiziert werden, wenn man die Bakterien über Tröpfencheninfektion appliziert.

Nach der Adhäsion an der Zelloberfläche wird L.monocytogenes durch Endozytose in die Zelle eingeschleust, zerstört unter der Einwirkung von Listeriolysin (Hly) die Endosomenmembran und wird so in das Zellzytosol freigesetzt [14]. In der Zelle angekommen, kann sich der Keim vermehren. Unter der Produktion weiterer Proteine bleibt das vollpathogene Bakterium nicht ortsständig, sondern bewegt sich aktiv fort. Die Fortbewegung wird durch die Ausnutzung einer Reihe von L.monocytogenes eigenen und einiger zelleigener Proteine bewerkstelligt [15, 16]. ActA wird auf der Zelloberfläche der L.monocytogenes exprimiert. Es bindet das zelluläre Protein VASP, das seinerseits die Brücke zur Anheftung von zellulären Aktin bildet. Im weiteren Verlauf bilden sich Aktinschweife aus, die das Bakterium an seiner Spitze tragen und es so durch die Zelle weiterbewegen. Trifft L.monocytogenes auf die Zellmembran, so entsteht eine Membranprotrusion, die bei benachbarten Zellen direkt in die Nachbarzelle hineinragt. Diese Ausstülpung wird dann von der Nachbarzelle endozytiert, so daß L.monocytogenes sich in der neuen Zelle innerhalb einer zweifachen Membran befindet. Die beiden Membranen werden unter Einwirkung von Hly und PlcB aufgelöst [17]. Am Ende dieses Vorganges hat L.monocytogenes auch die Nachbarzelle infiziert und der Infektionsprozeß beginnt von Neuem. So gelangt L.monocytogenes unter anderem in die sekretorischen Zellen des Euters wie der Kuh. Sekretierte Listerienproteine werden in der Milch nachweisbar, d.h. sie werden intrazellulär aus der laktierenden Zelle in die Milch abgegeben [18]. Zu diesen Proteinen zählen mit Hly (Listeriolysin) und IrpA (internalin related protein [19]) zwei Pathogenitätsfaktoren, die von L.monocytogenes im Wirt in großen Mengen produziert, sezerniert und in die Milch ausgeschieden werden [20].

Diese Kenntnisse des Infektionsprozesses haben es ermöglicht, L.monocytogenes genetisch so zu verändern, daß er fremde Proteine exprimiert. Beispiele für die Expression fremder Proteine in L.monocytogenes sind: Alkalische Phosphatase aus Escherichia coli, das Nucleoprotein aus dem Lymphochoriomeningitis virus (LCMV), das Nucleoprotein aus dem Influenza virus, das "major capsid protein" (L1) aus cottontail rabbit papillomavirus (CRPV) und das Gag Protein aus HIV Typ 1 [20 bis 27].

Neben Proteinen prokaryonten Ursprungs handelt es sich hierbei um virale Proteine, die normalerweise nicht innerhalb der eukaryonten Zelle produziert werden. Solche und ähnliche Fremdproteine prokaryonten und eukaryonten Ursprungs können von L.monocytogenes produziert werden, ohne daß eine eukaryonte Zelle dazu notwendig ist. Von L.monocytogenes produzierten Proteine werden in der Milch ausgeschieden.

Die Infektion durch Bakterien erfolgt durch spezifische Interaktionen von Liganden-Proteinen der Bakterien mit Rezeptor-Proteinen der Zielzellen. Hieran ist im Falle von L.monocytogenes die Internalinfamilie maßgeblich beteiligt; sie bestimmt im wesentlichen die Zellspezifität des Infektionsprozesses [28]. Zudem wird eine ActA abhängige Zellaufnahme diskutiert, die über Rezeptoren der Heparansulfatfamilie vermittelt wird [29]. Infiziert L.monocytogenes die Zelle, so kommt es nicht in jedem Fall zu einem vollen Infektionszyklus. Wird in L.monocytogenes Listeriolysin ausgeschaltet, so bleiben die Bakterien im Endosom stecken und die Infektion der "ersten Zelle" kommt nicht zu Stande. Bakterien, in denen das Protein ActA ausgeschaltet, inaktiv oder nicht mehr vorhanden ist, kommen in die erstinfizierten Zellen, bleiben aber dort stecken und können die Nachbarzellen nicht mehr infizieren [30, 31]. Bei der Ausschaltung der PclB ist der Keim nicht mehr in der Lage, sich in einer zweiten Zelle zu etablieren.

L.monocytogenes ist ein Bakterium, das durch eine Reihe von Antibiotika behandelt werden kann. Besonders geeignet sind Ampicillin und Penicillin (jeweils in Kombination mit Gentamycin). Als Alternative werden auch Erythromycin und Sulfonamide eingesetzt. In besonderen Fällen kommen Tetrazykline, Vancomycin oder Chloramphenicol zum Einsatz [32]. Entsprechende Behandlungsmöglichkeiten bestehen auch für andere Bakterien [8a] der Gattungen Aeromonas, Bartonella, Brucella, Campylobacter, Enterobacteriaceae, Mycobacterium, Renibacterium, Rhodococcus oder andere Bakterien, die mit dem genannten Bakterien genetisch oder biochemisch verwandt sind. Gelöst wird diese Aufgabe durch die eingangs genannte Verwendung von Bakterien in dem sogenannten TGC-Verfahren, wobei diese Verwendung dadurch gekennzeichnet ist, dass die Bakterien vital und in dem Organismus lebensfähig sind, dass sie entweder (a) voll pathogen sind oder (b) allenfalls derart attenuiert sind, dass eine Verhinderung der Apoptoseinduktion der eukaryonten Zelle und/oder eine Einschränkung der intrazellulären Motilität der Bakterien und/oder eine effiziente Eliminierung der Bakterien nach erfolgtem Gentransfer gewährleistet ist, oder (c) von Haus aus nicht pathogen und mit zusätzlichen Pathogenitätsfaktoren ausgestattet sind, die die Bakterien zu einer kontrollierten Infektion, zum Vordringen in Organe und Gewebe des Organismus und zur Übertragung von DNA in abgelegene Körperzellen befähigen, dass sie im Organismus ihrem typischen Infektionszyklus entsprechend und auf dem für sie typischen Infektionsweg zum Zielorgan und Zielgewebe vordringen und zur Übertragung der Fremd-DNA in abgelegene Körperzellen geeignet sind, dass der Infektionsweg natürlicherweise oder infolge gezielter genetischer Veränderungen gerichtet und örtlich begrenzt ist, wobei die gezielten genetischen Veränderungen insbesondere Gene betreffen, die die Vermehrung der Bakterien in den eukaryonten Zellen beeinflussen und/oder die Pathogenität der Bakterien im Organismus mindern und/oder das Überleben der Bakterien in der Umwelt nach einer Ausscheidung aus dem Organismus verhindern, und dass der Infektionszyklus durch den Einsatz von Antibiotika zeitlich begrenzbar und beendbar ist.

Vorteilhafte Weiterbildungen dieser Verwendung bzw. dieses TGC-Verfahrens sind in den Unteransprüchen genannt.

Gelöst wird diese Aufgabe außerdem durch ein Verfahren, bei dem Bakterien, die eine in einen episomalen Vektor integrierte Fremd-DNA aufweisen, deren Transkription und Expression unter der Kontrolle eukaryonter, regulatorischer Elemente steht, als Vehikel für einen Gentransport und Gentransfer in eukaryonten Zellen eines Organismus zur Induktion einer zielgerichteten somatischen Transgenität in diesen Zellen, Geweben oder Organen ausgenommen der Keimbahn des Organismus eingesetzt werden und in den infizierten und transgenen Zellen die Bildung des Fremd-Proteins initiieren, das anschließend aus der Zelle, dem Gewebe oder dem Organ isoliert und gereinigt wird, und wobei das Verfahren dadurch gekennzeichnet ist, dass die Bakterien vital und in dem Organismus lebensfähig sind, dass sie entweder (a) voll pathogen sind, oder (b) allenfalls derart attenuiert sind, daß eine Verhinderung der Apoptoseinduktion der eukaryonten Zelle und/oder eine Einschränkung der intrazellulären Motilität der Bakterien und/oder eine effiziente Eliminierung der Bakterien nach erfolgtem Gentransfer gewährleistet ist, oder (c) von Haus aus nicht pathogen und mit zusätzlichen Pathogenitätsfaktoren ausgestattet sind, die die Bakterien zu einer kontrollierten Infektion, zum Vordringen in Organe und Gewebe des Organismus und zur Übertragung von DNA in abgelegene Körperzellen befähigen, dass sie im Organismus ihrem typischen Infektionszyklus entsprechend und auf dem für sie typischen Infektionsweg das Zielorgan erreichen, dass der Infektionsweg natürlicherweise oder infolge gezielter genetischer Veränderungen gerichtet und örtlich begrenzt ist, wobei die gezielten genetischen Veränderungen insbesondere Gene betreffen, die die Vermehrung der Bakterien in den eukaryonten Zellen beeinflussen und/oder die Pathogenität der Bakterien im Organismus mindern und/oder das Überleben der Bakterien in der Umwelt nach einer Ausscheidung aus dem Organismus verhindern, und dass der Infektionszyklus durch den Einsatz von Antibiotika zeitlich begrenzbar und beendbar ist.

Die Bakterien, die eine Fremd-DNA tragen, welche in einem episomalen Vektor integriert und zur späteren Transkription und Expression vorbereitet ist, setzen bei der Infektion von Zellen, Geweben, einem Organ, dem ganzen Wirtsorganismus oder einem Organ in einer Kultur ihre genetische Information in die infizierte Einzelzelle frei und bewirken damit die Expression von Fremdprotein.

Vorteilhafte Weiterbildungen dieses Verfahren zur Gewinnung von Proteinen sind in den Unteransprüchen genannt.

Die erfindungsgemäß Verwendung der Bakterien bzw. das TGC-Verfahren kann entweder zur Gewinnung eines Fremdproteins eingesetzt werden, dient aber vorteilhaft auch zur somatischen Gentherapie, bei der die durch die bakterielle Infektion in den Wirtsorganismus eingeführte Fremd-DNA dort die Bildung eines dem Wirtsorganismus fehlenden Proteins veranlaßt oder durch die Erzeugung von einzel- oder doppelsträngiger Nukleinsäure die Bildung eines Proteins im Wirtsorganismus erhöht, vermindert oder verhindert. Dieses verfahren kann auf alle bekannten Nutztiere und auch auf den Menschen angewendet werden.

Handelt es sich bei dem infizierten Gewebe um das Ei eines Geflügeltieres, so wird das Fremdprotein im Ei produziert und kann aus diesem durch die bekannten Verfahren der Isolierung von Proteinen, zum Beispiel aus Hühnereiern, isoliert werden. Handelt es bei den infizierten Zellen um Zellen des Blutes, so kann durch parenterale Infektion der Zellen eine Verbreitung der Bakterien und mit ihnen der Fremd-DNA im injizierten Gesamtorganismus erzielt werden. Handelt es sich bei den Wirtstieren um Versuchstiere, deren infiziertes Organ ein Euter ist, so wird in der Milch des Versuchstieres das gewünschte Fremdprotein produziert, aus der das Fremd-Protein dann isoliert werden kann.

Das TGC-Verfahren wird im folgenden exemplarisch für das Bakterium L.monocytogenes besprochen. Es kann entsprechend aber auch bei allen intrazellulär wachsenden Bakterien angewendet werden, unter denen die Bakterien der Gattungen Aeromonas, Bartonella, Brucella, Campylobacter, Clostridia, Enterobacteriaceae (bei letzterer insbesondere Bakterien der Spezies Yersinia, Escherichia, Shigella, Salmonella), Legionella, Mycobacterium, Renibacterium, Rhodococcus oder Bakterien aus mit ihnen genetisch oder biochemisch verwandten Gattungen hervorzuheben sind, obwohl auch andere BakterienGattungen, die nicht pathogen sind und auch keinen intrazelulären Lebensstil haben, für das erfindungsgemäße Verfahren geeignet sind, sofern sie in einem eukaryonten Wirtsorganismus lebensfähig sind.

Es ist außerdem möglich, das TGC-Verfahren mit von Hause aus apathogenen Bakterien durchzuführen, die durch genetische Manipulationen mit zusätzlichen Faktoren ausgestattet werden, die ihnen den Zutritt zur Zelle ermöglichen. Zu einer derartigen "Aufrüstung mit Pathogenitätsfaktoren" können viele von Hause aus nicht intrazellulär wachsende Bakterien herangezogen werden, wie Bacillus subtilis, Lactobacillen, Pseudomonaden, Staphylokokken. Ein in diesem Sinn aufgerüsteter TGC-Sicherheitsstamm ist zum Beispiel Bacillus subtilis, der zusätzlich mit dem Listeriolysin aus L.monocytogenes ausgerüstet ist. Ein Beispiel für die Aufrüstung von apathogenen Bakterien zum TGC-Sicherheitsstamm wird mit der Ausstattung von L.innocua mit dem hly und/oder dem actA Gen aus L.monocytogenes im Beispiel 1 angegeben. Ein weiteres Beispiel ist E.coli K12 aufgerüstet mit dem Invasingen (inv) aus Yersinia pseudotuberculosis.

Das TGC-Verfahren wird in folgenden Schritten durchgeführt:

### a) Klonierung der TGC-(Fremd)-DNA:

Das TGC-Verfahren wird durch die Vorbereitung des L.monocytogenes Stammes im Labor eingeleitet. In einen geeigneten Vektor wird die cDNA für das herzustellende Fremdprotein insertiert. Die Einfügung der cDNA wird dabei in bekannter Weise so vorgenommen, daß die spätere Transkription und Expression im eukaryontischen Wirt sichergestellt ist. Soll das Protein von der Zelle sekretiert werden, so müssen die Vektoren passende, wirtszellspezifische Signalsequenzen enthalten. Beim Vektor kann es sich um einen eukaryonten Vektor handeln, zum Beispiel um pCMV der Firma Clontech oder um pCMD der Firma Invitrogen, beide käuflich zu erwerben. Als wesentliche Kriterien der Auswahlvektoren weisen diese eukaryonte Promotoren, Donor und Akzeptorstellen für das RNA-Splicing auf (fakultative Eigenschaft), sowie eine Polyadenylierungsstelle zum Beispiel aus SV40. Zur Vervielfältigung der DNA kann die Produktion der genetischen Konstrukte (im folgenden als TGC-DNA bezeichnet) in E. coli oder jedem anderem geeigneten Wirtsstamm nach Methoden vorgenommen werden. Die TGC-DNA muß zur primären Klonierung lediglich in die ausgewählten Bakterien eingebracht und dann später in den ausgewählten bakteriellen TGC-Sicherheitsstamm transferiert werden können. Der Transfer in L.monocytogenes kann mit den verschiedenen, wohlbekannten Methoden des Gentransfers von isolierter DNA (Transformation, Elektroporation etc.) oder durch die Vorgänge der Konjugation und der Transduktion direkt oder mittelbar von Bakterium zu Bakterium vorgenommen werden.

### b) TGC-Sicherheitsstämme als Rezipienten der TGC-DNA:

Als Rezipient der TGC-DNA werden besondere L.monocytogenes Wirtsstämme - oder aber andere TGC-Ammen, die wie L.monocytogenes intrazellulär vitale Bakterien (z.B. Yersinien), oder ins Endosom-eindringende (z.B. Samonellen) oder aber mit zusätzlichen bakteriellen Faktoren "aufgerüstete", ansonsten nicht pathogene Bakterien (z.B. Escherichia coli oder L.innocua) darstellen - eingesetzt, die folgende Eigenschaften, einzeln oder in Kombination, erfüllen:
- (A.1): sie eignen sich als Empfänger fremder DNA (genetische Manipulierbarkeit);
- (B.1): sie tragen Mutationen, die Gene betreffen, ohne die ein Überleben der Bakterien in der Außenwelt, also außerhalb eines Wirtes, zum Beispiel bei niedrigerer Temperatur, unmöglich ist (sicherheitsrelevante Eigenschaft);
- (B.2): es sind attenuierte Wirtsstämme, bei denen ein Teil ihrer Pathogentitätsfaktoren deletiert oder inaktiviert ist, so daß sie nicht mehr die volle Pathogenität der Wildtypstämme entfalten (Attenuierung);
- (C.1): sie sind "genetische Krüppel", die durch gezielte, vom Experimentator eingeführte Stoffwechseldefekte nur auf definierten, artifiziellen Medien zur Anzucht gebracht werden können, aufgrund dieser Defekte aber in der Zelle und vor allem im Ganztier nicht mehr wachsen, sich also nicht vermehren können ("endogener Suizid");
- (C.2): sie induzieren ihre Aufnahme in Endosomen und werden in diesen zellkompartimenten aufgelöst (Infektion über Endosomen)
- (C.3): sie werden von professionellen Phagozyten in Phagolysosomen aufgenommen, können diese Zellkompartimente jedoch auflösen (d.h. überwinden) (Infektion über Phagolysosomen)
- (C.4): die Bakterien tragen Suizidgene, die erst nach dem Eindringen in die Wirtszelle konditional aktiviert werden, so daß die Bakterien sich selbst umbringen ("exogener Suizid")
- (D.1): sie sind durch Antibiotikabehandlung des späteren animalen Wirts zu eliminieren (Abtötung durch Antibiose).

Bei Punkt A.1 handelt es sich um eine allgemeine Eigenschaft der Bakterien, ohne die keine der angesprochenen genetischen Manipulationen möglich wäre.

Unter den Punkten B.1 und B.2 sind Veränderungen zusammengefaßt, die die Anwendung der Bakterien sicherer machen. Bakterien mit diesen Veränderungen können sich bei Ausscheidung in die Umwelt nicht mehr vermehren, bzw sind attenuiert (B.1), weisen eine geringeres pathogenes Potential auf (B.2). Die Veränderung von Bakterien gemäß Unterpunkt B.2 hat auch Einfluß auf die Freisetzung der Fremd-DNA in die Zelle (siehe Unterpunkte C.2 + C.3).

Bei den Unterpunkten C.1 - C.4 handelt es sich um genetische Veränderungen von Bakterien, die die Freisetzung der Fremd-DNA in die animale Zelle maßgeblich bestimmen. Unter den Punkten C.3 - C.4 sind Infektionswege aufgezeigt, die für Bakterien, die weiter unten unter den Beispielen zusammengefaßt werden, als Weg der Übertragung der Fremd-DNA in das Zytosol der animalen Zelle identifiziert wurden.

Der unter (D.1) aufgeführte Eingriff erlaubt die gezielte Abtötung der Bakterien. Dadurch wird die Freisetzung der Fremd-DNA aus den Bakterien bewirkt, die Therapie mit Antibiotika ist aber auch ein sicherheitrelevanter Aspekt.

Über die Veränderungen und Eingriffe gemäß C.1 - C.4 und auch B.2 und D.1 wird die Freisetzung der rekombinanten DNA in die Zelle erst ermöglicht.

Stämme mit diesen Eigenschaften (einzeln oder in Kombination) werden als TGC-Sicherheitsstämme bezeichnet.

### c) Optimierung der TGC-Amme auf das Zielorgan des TGC-Verfahrens:

Die TGC-DNA, die für das herzustellende Fremdprotein kodiert, wird in den TGC-Sicherheitsstamm durch Transformation, Konjugation oder Transduktion überführt. Die so erhaltenen Stämme werden nachfolgend als TGC-Amme bezeichnet. Die Amme versorgt (füttert) den TGC-Wirt mit der DNA und induziert damit die somatische Transgenität. Damit während des TGC-Verfahrens das gewünschte Fremdprotein optimal exprimiert wird, sollte sich das Gen vorteilhafterweise unter der Kontrolle von Promotoren und anderen regulatorisch wirkenden Sequenzen befinden, die aus dem vorher ausgewählten Zielorgan des TGC-Verfahrens stammen oder für das Zielorgan optimiert sind, zum Beispiel Euter-spezifische Promotoren und Sekretionssignale.

### d) Infektion des Wirtsorganismus mit der TGC-Amme:

Durch die Anzucht der TGC-Amme wird sie in vitro in einem Kulturmedium vermehrt und für die Durchführung des TGC-Verfahrens in einem ausgewählten Wirtsorganismus vorbereitet. Alternativ kann die TGC-Amme auch im dem Wirtsorganismus (Mensch oder Tier, im folgenden TGC-Wirt genannt), durch in vivo Anzucht vermehrt. Als Vorbereitung für die Infektion wird die TGC-Amme in einer an den TGC-Wirt adaptierten, nicht bakteriziden Lösung, einem Puffer oder einer anderen physiologischen Flüssigkeit aufgenommen. Die Flüssigkeit wird dem TGC-Wirt, zum Beispiel dem laktierenden Säugetier, wenn das Euter somatisch transgen gemacht werden soll, verabreicht. Dies kann entweder peroral durch Trinken der Flüssigkeit oder aber durch Zuführung über eine Magensonde, den Anus oder eine andere Körperöffnung erfolgen. Als Alternative kommt die Gabe der TGC-Amme durch Injektion in Betracht, die intravenös, intramuskulär direkt in das Zielorgan oder vorzugsweise intraperitoneal erfolgt. Als weitere Alternative kann die Infektion durch Bildung eines Aerosols und anschließende Inhalation der Tröpfchen erfolgen.

Der TGC-Wirt (Mensch oder Nutztier: Kuh, Pferd, Ziege, Schaf, Schwein, Hase, Geflügel etc.) kann mehrmals mit gleichen oder heterologen Transgenen infiziert werden. Durch mehrmalige Infektion mit unterschiedlicher DNA, die zum Beispiel für mehrere Enzyme eines Biosyntheseweges kodieren, können auf diese Weise ganze Enzymkaskaden in dem TGC-Wirt etabliert werden. Dadurch läßt sich auch die biochemische Expression multigener Proteine erreichen.

### e) Organ- und Zellspezifität der Infektion:

Der weitere Weg der TGC-Amme im Organismus ist zunächst durch den natürlichen Infektionsweg bestimmt. Die TGC-Amme erreicht auf dem für sie typischen und vom jeweiligen Bakterium gesteuerten Weg das Zielorgan. Trägt die TGC-Amme im Fall von L.monocytogenes genetisch unveränderte Internaline, so ist das Euter unter den Zielorganen. Genetisch veränderte Internaline erlauben die Infektion anderer Organsysteme. Seinem Infektionszyklus entsprechend dringt die TGC-Amme in die Zellen ein und erscheint im Zytoplasma. Da sie genetisch defekt ist, kann die TGC-Amme sich dort nicht weiter vermehren, sie begeht "endogenen Suizid" (siehe oben, C.1 unter Aufzählungspunkt (b)). Bei der Zellinfektion hat die TGC-Amme die wirtsfremde TGC-DNA in ihrem Gepäck in die Zelle eingeschleust. Die Übertragung der Fremd-DNA in die Zelle kann aber auch durch "exogenen Suizid" (siehe oben, C.4 unter Aufzählungspunkt (b)) oder durch "Abtötung" der Bakterien durch gezielte Antibiose (siehe oben, C.3 unter Aufzählungspunkt (b))ausgelöst werden. In diesen drei Fällen stirbt die Bakterienzelle, die die Fremd-DNA in sich trägt, innerhalb der animalen Zelle und entläßt dabei die Fremd-DNA ins Zytoplasma. Schließlich kann die Übertragung der Fremd-DNA auf die animale Zelle auch durch gezielte Infektion der Zellen unter Ausbleiben der Lyse der Endosomen erreicht werden. Hierbei wird die Fremd-DNA der animalen Zelle durch Lyse der Bakterien innerhalb der Endosomen zugänglich.
In jedem der angesprochenen Fälle steht die in die Zelle übertragene DNA nun als Template zur Produktion des gewünschten Fremdproteins zur Verfügung. Die Nukleinsäure kann aber auch direkt therapeutisch wirken, zum Beispiel durch Generierung einer anti-sense RNA. Die so manipulierten Zellen, Gewebe oder Organe werden im Verlauf der Infektion somatisch transgen.

### f) L.monocytogenes induzierte Proteinproduktion in der Milch von Säugetieren:

Nach Ausführen des TGC-Verfahrens - zum Beispiel mit TGC-Ammen wie L.monocytogenes aber auch anderen intrazellulär vitalen Bakterien (z.B. Yersinien), oder ins Endosom eindringenden (z.B. Samonellen) oder aber mit zusätzlichen bakteriellen Faktoren aufgerüsteten, ansonsten nicht-pathogenen Bakterien (z.B. Escherichia coli oder L.innocua) - wird das Protein wird in der laktierenden Zelle gebildet und mit den anderen Produkten der Zelle in die Milch ausgeschieden. Werden mehrere Tiere mit unterschiedlicher Fremd-DNA im TGC-Verfahren somatisch transgen gemacht, so können durch Sammeln der Milch jedes einzelnen TGC-Wirtes (Melken) die unterschiedlichen Proteine voneinander getrennt produziert werden.

Aufgrund der Eigenschaften der TGC-Amme tauchen in der Milch keine L.monocytogenes (TGC-Ammen, d.h. Bakterien) auf. Sollte dies dennoch der Fall sein, so können die Bakterien mit allen dem Fachmann bekannten Methoden eliminiert werden, zum Beispiel durch Behandlung mit Antibiotika. Die Tiere (oder auch der Mensch) sind nach der Durchführung des "targeted genetic conditioning" (TGC) frei von lebensfähigen, gentechnisch veränderten Organismen und unterliegen damit keinen weiteren Sicherheitsbestimmungen. Der TGC-Wirt gibt die in ihn durch das TGC-Verfahren eingetragene genetische Information an die nachkommenden Zellen im Rahmen der üblichen Zellteilung weiter. Auf Abkömmlinge des TGC-Wirts wird die Information jedoch nicht übertragen, da die TGC-DNA sich nicht in der Keimbahn des TGC-Wirtes befindet. Das Umgehen (d.h. Auslassen)der genetischen Manipulation der Keimbahn des Gesamtorganismus und die gezielte Proteinproduktion im vorbestimmten Organ oder Gewebe des animalen Wirts (Tier, ausgenommen Mensch) stellt den innovativen und neuen Aspekt des erfindungsgemäßen Verfahrens dar.

### g) Infektionen von Geweben durch L.monocytogenes

Das Blut ist ein Gewebe, dessen erfindungsgemäße genetische Veränderung durch das TGC-Verfahren exemplarisch beschrieben wird. Die Zellen des Blutes eignen sich besonders für das TGC-Verfahren. Die Infektion von Zellen des Blutes läßt sich außerhalb des Körpers vornehmen. Die zu erzielende somatische Transgenität der Zellen kann ebenfalls außerhalb des Wirtes überprüft werden. Im Fall der attenuierten, auxotrophen Bakterien lassen sich die zum Wachstum der Zellen notwendigen Substanzen - als Beispiel für Auxotrophie dient hier die Diaminopimelinsäure - dem Medium zufügen und so der Zeitraum der Lebensfähigkeit der Bakterien entsprechend dem Versuchsziel steuern. Durch anschließende Lyse der animalen Zellen kann dann überprüft werden, ob die intrazellulären Bakterien noch vital sind.

Zur Reimplantation in den Empfängerorganismus werden schließlich die transfizierten Zellen verwendet, die eine genau bekannte Menge an vitalen Bakterien enthalten. Im Einzelfall können dies so große Mengen an Bakterien sein, daß im Organismus weitere Organe infiziert werden. In anderen Fällen wird durch die Eliminierung vitaler Bakterien in vitro vor der Reimplantation in den TGC-Wirt die Transgenität gezielt auf das Gewebeblut beschränkt.

Die Reimplantation und damit verbunden die Disseminierung der transgenen Zellen mit oder ohne vitale Bakterien erlaubt die somatische Gentherapie von Zellen im Wirt, der in diesem Fall auch ein Mensch sein kann.

Das TGC-Verfahren ermöglicht es aber auch, extrakorporal Proteine zu produzieren. Dazu werden die TGC-Ammen in Eier von Geflügeltieren injiziert. Geeignete Techniken hierfür sind in der Impfstoffherstellung bei viralen Erregern bereits Stand der Technik. Im Verlauf der Bebrütung der Eier werden die Zellen im Ei infiziert, somatisch transgen und produzieren dann das Fremdprotein. Aus dem Ei läßt sich nach bekannten Verfahren das Fremdprotein aufreinigen. Bei dieser Form des TGC-Verfahrens bleibt die TGC-Amme in allen Phasen der Anwendung unter Laborbedingungen kontrollierbar. Die Menge des zu produzierenden Proteins hängt nur von der Injektion einer entsprechend großen Anzahl von Eiern ab.

### h) Einsatz des TGC-Verfahrens zur somatischen Gentherapie:

Es gibt noch keine etablierte Form der somatischen Gentherapie. Derzeit wird die zur Transfektion eingesetzte Nukleinsäure im Inneren von Viren oder eingepackt in Liposomen vor Einflüssen der Außenwelt geschützt.

Viren haben den Nachteil, daß sie nur eine begrenzte Aufnahmefähigkeit haben und daß bei hohen Infektionsdosen mit der Entwicklung ihrer vollen zytopathischen Effekte zu rechnen ist [32a]. Sie induzieren Immunreaktionen und können so selber angegriffen und zerstört werden. Einige Viren werden durch Serum inaktiviert und werden dann für die Gentherapie unbrauchbar. Hier ist besonders die mehrfache Gabe von Viren zur Gentherapie zu nennen, in deren Verlauf die Immunantwort des Wirtes angestoßen wird. Die Bildung einer gezielten gegen die Viren gerichteten Abwehr stellte sich zuletzt als ein wesentliches Problem der Verwendung von Viren im Rahmen der Gentherapie heraus.

Beim Einsatz von Liposomen ist die toxische Wirkung der Lipide zu berücksichtigen, die entzündliche Reaktionen auslöst.

Im Fall der in vivo Therapie gibt es noch erhebliche Defizite auf dem Weg zu Anwendung der bisher benutzten Systeme des Gentransfers. Für diese Form der Therapie wird gefordert [32b]:
(i) die Resistenz des Vektors gegen Abbau nach der in vivo Gabe im Körper,
(ii) die Gewebsspezifität, d.h. das gezielte Ansteuern des zu therapierenden Gewebes (Organs) und
(iii) die Sicherheit, womit die Unschädlichkeit der nicht zu behandelnden Organe gemeint ist [32b].

Die in dieser Patentanmeldung aufgeführten Bakterien, die hier als Vehikel zum Gentransport und Gentransfer eingesetzt werden können sollen, sind idealerweise für den Gentransfer geeignet. Die Bakterien sind auf ihren jeweiligen Wirt bestens adaptiert und können in ihm ohne äußere Eingriffe, wie Antibiotika-Therapie für eine ausreichende Zeit überleben. Sie induzieren auf definierten Infektionswegen spezifische Erkrankungen und zeigen dabei zum Teil eine ausgeprägte Organotropie. Sie können erhebliche Fremd-DNA Mengen aufnehmen (z.B. natürlich vorkommende Plasmide haben Größen von mehreren 100 Kilobasen), so daß nicht nur c-DNA's sondern sogar größere Bereiche eines Chromosoms übertragen werden können. Schließlich können sie sicher eingesetzt werden, insbesondere wenn es sich um "Krüppelbakterien" handelt, wie sie oben beschrieben worden sind. Die genetischen Defekte der TGC-Amme in Kombination mit der vorgegebenen Antibiotika-Empfindlichkeit garantieren eine effiziente Eliminierung der Bakterien, nachdem sie ihre Aufgabe, den DNA-Transfer in die eukaryonte Zelle erfüllt haben.

### Beispiel:

Als Beispiele für eine somatische Gentherapie sind hier aufgeführt:
- Die Therapie der cystischen Fibrose (CF): Dazu muß der Keim dem zu therapierenden Patienten durch Inhalation verabreicht werden. Bei dem Bakterium handelt es sich vorzugsweise um einen Keim, der über Tröpfcheninfektion übertragen wird. In dem Bakterium befindet sich das CFTR-Gen, das den bei der CF maßgeblichen Defekt kurieren kann. Das Bakterium dringt in die Säulenzellen des Luftweges (airway lumen-facing columnar cells) ein und transfiziert diese mit der CFTR-DNA integriert in den TGC-Vektor. Die Zellen werden somatisch transgen, der Defekt wird kuriert.
- Durch somatische Gentherapie mit dem humanen β-Globulingens kann die β-Thalassaemie behandelt werden. Dazu werden ex vivo Stammzellen der haematopoetischen Reihe mit einem TGC-Sicherheitsstamm infiziert, der das β-Globulingen auf die Stammzelle überträgt. Durch Behandlung der Zellen in der Zellkultur wird das infizierende Bakterium eliminiert und die transgene Zelle für die Rückübertragung auf den Menschen vorbereitet. Diese Übertragung erfolgt durch intravenöse Gabe.
- Bei der Therapie des Hurler Syndroms werden primitive CD34-positive Zellen des Knochenmarks mit dem α-L-Iduronidase-Gen transfiziert. Der Weg der Gentherapie und der Rückübertragung der Zellen auf den Patienten entspricht dem im vorherigen Abschnitt beschriebenen Weg.
- Bei der Gentherapie der Fanconi Anaemie wird das Gen der Fanconi Anaemie Komplementationsgruppe C (FACC) zur somatischen Gentherapie eingesetzt. Zielzellen der Infektion mit der TGC-Amme sind hierbei erneut CD34-positive Zellen des Knochenmarks.

### i) Nachweis des Erfolges des TGC-Verfahrens

Der DNA-Transfer wird in der Maus bereits innerhalb der ersten 24 Stunden sichtbar, d.h. lange bevor eine spezifische Immunantwort gegen das Bakterium entstanden sein konnte. Gezeigt wurde dies durch die Produktion von β-Galactosidase oder von dem fluoreszierenden Protein EGFP in Zellkulturen innerhalb von 24 Stunden. Der im Rahmen der Infektion zusätzlich auftretende "mitogene Effekt der Bakterien" begünstigt die Etablierung der DNA in der TGC-Zelle und ist damit erwünscht und für den Erfolg des TGC-Verfahrens vorteilhaft.

Zusammenfassend ist deshalb festzustellen, daß der Einsatz von Bakterien zur somatischen Gentherapie sicherer ist als die Gentherapie mit viralen Systemen. Die bakterielle Infektion kann gerichtet und örtlich begrenzt werden. Das Wachstum und damit die floride Infektion durch die Bakterien kann durch Ausschalten bestimmter bakterieller Faktoren verhindert werden. Außerdem kann das Wachstum der Bakterien in der eukaryonten Zelle genau beeinflußt und generell verhindert werden. Schließlich ist die Beendigung der bakteriellen Infektion durch den Einsatz von Antibiotika jederzeit möglich, d.h. die Infektion läßt sich örtlich, zeitlich und effektiv begrenzen.

Im folgenden wird am Beispiel von L.monocytogenes die Erfindung detailliert beschrieben:

### Beispiel 1: Die Herstellung von TGC-Sicherheitsstämmen

Die L.monocytogenes Sicherheitsstämme werden durch gezielte genetische Veränderungen von primär pathogenen L.monocytogenes Stämmen hergestellt. Dabei werden mehrere Ebenen der Sicherheit parallel zueinander etabliert. Damit wird verhindert, daß durch Rückmutationen die Vitalität oder Pathogenität wieder entstehen können. Die Mutationen betreffen Gene, die (1) das Überleben der Bakterien in der Zelle beeinflussen, (2) die die Pathogenität der Bakterien im TGC-Wirt mindern und (3) die das Überleben der Bakterien in der Umwelt nach einer möglichen Ausscheidung verhindern.

### a) Erste Ebene der Sicherheit - Sicherheitsrelevante Eigenschaft: Überleben in der Umwelt (siehe oben, B.1 unter Aufzählungspunkt (b))

Die TGC-Ammen können dem TGC-Wirt entweder durch Injektion oder durch perorale Gabe appliziert werden. Bei peroraler Gabe kann es zu einem Überangebot an Bakterien und dadurch zur Ausscheidung von Bakterien kommen, die vom Organismus nicht aufgenommen werden. Damit diese ausgeschiedenen Bakterien keine Chance haben, in der Umwelt zu überleben, können die TGC-Sicherheitsstämme zusätzliche Mutationen enthalten, die das Wachstum der Bakterien in der Umwelt verhindern.

Als ein Beispiel hierfür wird das Ausschalten des cspL-Gens (cold shock protein der Listerien) angeführt. Das hat zur Folge, daß die Bakterien bei Temperaturen unter 20°C nicht mehr wachsen können. Das Wachstum und die Infektionsfähigkeit bei 37°C sind nicht beeinträchtigt, werden allerdings bei gleichzeitigen Mutationen gemäß a) und b) zusätzlich moduliert. Das cspL-Gen, das in den erfindungsgemäß eingesetzten Sicherheitsstämmen deletiert ist, ist im Sequenzprotokoll als SEQ. ID No. 2 dargestellt. Ein entsprechender cspL-deletierter Stamm ist unter der Bezeichnung L.monocytogenes EGD Delta cspL1 bei der DSM unter der Nr. 11883 hinterlegt worden.

Die erfindungsgemäß eingesetzten TGC-Sicherheitsstämme können nur auf speziellen Wachsstumsmedien angezüchtet werden. Die Wachstumstemperatur muß dabei über 37°C betragen, unter 20°C ist ein Wachstum unmöglich. Die Bakterien besitzen eine eingeschränkte Pathogenität und vermögen nur noch in begrenzte, eng umschriebene Areale des TGC-Wirtes vorzudringen. Damit wird die Sicherheit des Systems für Mensch und Umwelt garantiert. Die TGC-Ammen sind außerhalb der artefiziellen Medien, hier speziell in der Wirtszelle, nicht mehr wachstumsfähig. Diese eingeschränkte intrazelluläre Lebensfähigkeit ist gleichzeitig eine Voraussetzung für die Freisetzung der TGC-DNA in der Wirtszelle und damit für die Induktion der somatischen Transgenität im TGC-Verfahren.

### b) Zweite Ebene der Sicherheit - Attenuierung: verminderte Pathogenität (siehe oben, B.2 unter Aufzählungspunkt (b))

Die zweite Ebene der Attenuierung der TGC-Sicherheitsstämme umfaßt Mutationen in den Pathogenitätsfaktoren. Durch gezielte Mutationen in definierten Faktoren wird die Pathogenität der Bakterien abgeschwächt, die Apoptoseinduktion der infizierten Wirtszelle verhindert und gleichzeitig die Immunreaktion in eine gewünschte Richtung dirigiert. Die Mutationen schränken die intrazelluläre Motilität der Bakterien und damit ihre Ausbreitung in sekundäre Zellen ein. Dadurch wird die Infektion unter Beibehaltung der Behandelbarkeit durch bekannte Antibiotika auf die ausgesuchten Zielzellen begrenzt.

Aus Sicherheitserwägungen ist es wünschenswert, die intrazelluläre Ausbreitung der TGC-Amme nach der Infektion einzuschränken oder gar zu verhindern. Das genaue Wissen über die intrazelluläre Lebensweise und die Motilität der obengenannten Bakterien ermöglicht es, definierte, stabile Mutanten mit verminderter Fähigkeit zur Infektion des TGC-Wirtes zu erzeugen.

Bei L.monozytogenes betreffen die in diesem Sinne attenuierten Mutationen zum Beispiel das hly-Gen mit der Folge der Blockade der Infektion der ersten Zelle. Als ein Beispiel für die Ausschaltung dieses Pathogenitätsfaktors ist der Stamm L. monocytogenes EGD Hly_{D491A} hinterlegt worden und hat die Hinterlegungsnummer DSM 11881 erhalten.

Ein anderes Beispiel für die Verminderung der Pathogenität von L.monocytogenes sind Mutationen im actA-Gen oder die Deletion von Regionen, die für die Interaktion zwischen actA und dem Wirtszellprotein VASP erforderlich sind, mit der Folge, daß die intrazelluläre Motilität blockiert ist. Schließlich gibt es auch Mutationen des plcB-Gen, wodurch den Bakterien die Möglichkeit genommen wird, sich in eine zweite Zelle hinein auszubreiten. Der hinterlegte Stamm L. monocytogenes EGD Delta actA Delta plcB ist ein Beispiel für eine doppelte Mutation, in der sowohl das actA-Gen als auch das plcB-Gen ausgeschaltet sind. Er trägt die Hinterlegungsnummer DSM 11882.

Außerdem ist es auch möglich, in L.monocytogenes das Wildtyp-Listeriolysin-Gen gegen ein mutiertes Allel auszutauschen. Dann werden die Eigenschaften des Listeriolysin, sowohl eine Apoptose in verschiedenen Wirtszellen zu induzieren als auch und eine starke T-Zellvermittelte Immunantwort zu generieren, eingeschränkt.

### c) Überleben in der Zelle: - Endogener Suizid: 3. Ebene der Sicherheit (siehe oben, C.1 unter Aufzählungspunkt (b))

Allgemein zeichnen sich die für das TGC-Verfahren attenuierten Bakterien durch definierte Deletionen in den Genen aus, die für die Biosynthese integraler bakterieller Komponenten essentiell sind. Die ausgewählten auxotrophen Bakterien eignen sich als TGC-Amme, denn als attenuierte Bakterien können sie fremde DNA in die Zelle transportieren. Da die Bakterien jedoch in den Zellen von essentiellen "Wachstumsfaktoren" abgeschnitten sind, lysieren sie sponaten und setzen dabei die TGC-DNA in der Zelle frei.

Als TGC-Sicherheitsstämme werden L.monocytogenes eingesetzt, die genetisch derart verändert sind, daß sie die Zelle zwar noch infizieren, sich aber in der Zelle nicht mehr vermehren können. Dies wird u.a. durch die Inaktivierung des dapE-Gens in L.monocytogenes erreicht. Listerien sind Gram-positive Bakterien, die ebenso wie die Gram-negative Bakterien zur Vernetzung der Zellwand meso-Diaminopimelinsäurederivate (DAP) benötigen. Die Biosynthese von Diaminopimelinsäure ist daher für die Bildung der bakteriellen Zellwand essentiell. DAPauxotrophe Bakterien unterliegen der spontanen Lyse, wenn diese Aminosäure im Kuluturmedium nicht mehr angeboten wird. Die Enzyme, die bei der DAP-Synthese im Bakterium beteiligt sind, sind in Säugerzellen nicht vorhanden. In TGC-Sicherheitsbakterien sind eben diese Enzyme deletiert oder durch Insertionen oder auf eine andere Weise inaktiviert. Das dapE von L.monocytogenes, das in den erfindungsgemäß eingesetzten Sicherheitsstämmen inaktiviert wurde, ist im Sequenzprotokoll als SEQ. ID No. 1 dargestellt. Zur genetischen Manipulation des dapE Gens in L.monocytogenes mußte dessen Sequenz bestimmt werden, den entsprechende Gene z.B. aus E.coli weisen lediglich eine ca 30%ige Homologie zur Sequenz des SEQ ID No. 1 Protokoll auf.

Die dieses oder anderer Gene des DAP-Biosyntheseweges beraubten Bakterien, sog. DAP-Mutanten, können weder innerhalb noch außerhalb des Wirtes wachsen. Dazu benötigen sie den Zusatz von großen Mengen an DAP (1mM) zum Wachstumsmedium. Fehlt es an DAP, kann das Bakterium weder im TGC-Wirt noch außerhalb des TGC-Wirts überleben. Damit bieten diese DAP-Mutanten sowohl Sicherheit vor einer bakteriellen Infektion des TGC-Wirtes als auch Sicherheit vor einer Infektion anderer Organismen im Falle der Freisetzung eines derartigen Stammes in die Außenwelt.

Ein Eingriff ins Genom von Salmonellen mit entspecehnder Auswirkung (Schaffung einer auxotrophen Mutante) stellt die Deletion (oder Blockade oder Mutagenese) des aroA Gens dar, das zur Synthese aromatischer Aminosäuren essentiell ist. Aus dem Salmonellen Impfstamm (zugänglich bei der amerikanischen Stammsammlung unter ATCC14028) läßt sich durch genetische Manipulation mit Techniken, die dem Fachmann bekannt sind, und in Kenntnis des aroA Gens (Genebank Annahme-Nummer M 10947) eine Mutante gewinnen, die analog den hier für Listerien beschriebenen rekombinanten Bakterien als TGC-Sicherheitsstamm fungieren kann. Die Freisetzung der Fremd-DNA erfolgt, wie für den oben beschriebenen L.monocytogenes delta dapE Stamm, durch Absterben der Bakterien nach Aufnahme in die Zelle. Anderes als L.monocytogenes vermögen Salmonellen nicht in das Zytoplasma der Zelle zu gelangen. Die Freisetzung der Fremd-DNA erfolgt in diesem Fall aus den Endosomen ins Zellinnere.

Es sind auch noch andere attenuierte Mutationen von L.monocytogenes bekannt, in denen die Biosynthese von Nukleinsäuren, Aminosäuren, Zuckern oder anderen Zellwandbausteinen blockiert ist [33 bis 35]. Gleiches kann auch durch Mutationen in regulatorischen Genen erreicht werden, die für den intrazellulären Lebensstil der Bakterien essentiell sind. Beispielhaft für ein derartiges Gen ist phoP von Salmonella typhimurium [36].

Die hier aufgeführten Beispiele für L.monocytogenes sind auf andere intrazellulär vitale Bakterien oder Bakterien übertragbar, die erst durch Aufrüstung mit Pathogenitätsfaktoren zu intrazellulären Erregern gemacht werden. Dies gilt insbesondere für Bakterien der Gattungen Aeromonas, Bartonella, Brucella, Campylobacter, Clostridia, Enterobacteriaceae (besonders E.coli, Salmonellen, Shigellen, Yersinien), Mycobaterium, Renibacterium und Rhodococcus. Ein in diesem Sinn aufgerüsteter TGC-Sicherheitsstamm ist zum Beispiel Bacillus subtilis, der zusätzlich mit dem Listeriolysin aus L.monocytogenes ausgerüstet ist.

Eine wichtige Voraussetzung für die Übertragung von DNA selbst in "abgelegene" Körperzellen ist der Schutz der DNA auf dem Weg bis zur Zielzelle oder dem Zielgewebe oder dem Zielorgan. Die Fähigkeit intrazellulär vitaler Bakterien wie L.monocytogenes zur intrazellulären Ausbreitung ist eine ideale Eigenschaft, Gene in abgelegene Zellen, in tiefere Gewebe und Organe zu transportieren. Nach erfolgreichemn Transfer der TGC-DNA in die Zielzelle verstirbt der Bote, die TGC-Amme, in Folge der Attenuierung (B.1), der Induktion von Auxotropien (B.2), des endogenen Suizids (C.1), der Infektion über Endosomen (C.2), der Infektion über Phagolysosomen (C.3), des exogenen Suizids (C.4) oder der Antibiotikatherapie (D.1).

### Beispiel 2: Freisetzung der Fremd-DNA in der animalen Zelle (synonym: Gewebe; Organ)

### a) Infektion über Endosomen: Transfer des Expressionsplasmids ohne Befreiung der Bakterien aus dem Endosomvesikel (siehe oben, C.2 unter Aufzählungspunkt (b))

Es wurde getestet, ob Bakterien in der Lage sind, ihre Plasmid-DNA ins Zytoplasma infizierter Wirtszellen zu übertragen, ohne daß sie sich vorher aus dem Endosomvesikel befreit haben müssen. Dazu wurde die Fähigkeit der L. monocytogenes Dhly Mutante, die nicht mehr aus dem Endosom austreten kann, untersucht, als übertragendes Bakterium für den DNA-Transfer zu fungieren. Als zu übertragende Fremd-DNA wurde EGFP gewählt, ein fluoreszierendes Protein, das unter der Kontrolle eines CMV-Promotors kloniert war. Als Maß für die erfolgreiche Übertragung der Fremd-DNA - d.h. als Maß für die Transfektion der eukaryonten Zelle - wurden 10.000 Zellen im FACS-Scanner nach Infektion mit den jeweiligen L.monocytogenes Stämmen auf das Vorkommen der EGFP-ausgelösten Fluoreszenz untersucht. Die Anzahl ist in Tabelle 1 in % der Gesamtzahl der gemessenen eukaryonten Zellen ausgedrückt. Bei den Experimenten diente der L. monocytogenes Wildtyp-Stamm EGD als positiv Kontrolle. Geprüft wurde auch eine isogener, nicht invasiver ADInlAB Stamm. Die Aussagen, die mit diesen Bakterien gewonnen wurden, haben Allgemeingültigkeit und sind auf andere Bakterien übertragbar.

Die Ergebnisse sind in Tabelle 1 zusammengefaßt und zeigen, daß die Dhly Mutante in Bezug auf den DNA-Transfer vom Bakterium in die eukaryonte Zelle genauso effizient ist, wie der L. monocytogenes Wildtyp-Stamm. Der L.monozytogenes DInlAB Stamm ist als Vehikel für DNA-Transfer in die hier angegebenen Zellen nicht (PtK2) oder aber wesentlich schlechter (Hep-2) geeignet. Die Experimente zeigen weiterhin, daß die aktive Aufnahme der Bakterien durch die eukaryonte Zelle (in diesem Fall keine professionellen Phagozyten) die Voraussetzung für die Transfektion der Zellen ist. Die Anheftung der Bakterien wird durch die Interaktion zwischen bakteriellen Internalinen (InlA und/oder InlB) und den Rezeptoren der animalen Zellen bewirkt. Die Experimente des folgenden Beispiels zeigen, daß Internaline zur Aufnahme von Bakterien in professionelle Phagozyten nicht notwendig sind.

| **Zelllinie** | **Ursprung** | **L.monocytogenes-Stamm** | **Transfizierte Zellen in %** |
|---|---|---|---|
| PtK2 | Kangaroo rat kidney | Wildtyp EGD | 1.71 |
| | | Δhly | 1.78 |
| | | ΔinlAB | 0 |
| Hep-2 | human larynx carcinoma | Wildtyp EGD | 4.58 |
| | | Δhly | 4.31 |
| | | ΔinlAB | 0.24 |

### b) Infektion über Phagolysosomen: Aufrüstung nicht pathogener Stämme zum TGC-Sicherheitsstamm:(siehe oben, C.3 unter Aufzählungspunkt (b))

Das im folgenden für L. innocua dargestellte Beispiel ist exemplarisch und kann auf andere nicht pathogene Bakterien (z.B. Escherichia coli) übertragen werden. Die auszuführenden Eingriffe in die genetische Ausstattung solcher Bakterien entsprechen den hier für L.innocua angeführten.

Ein nicht pathogener L. innocua Stamm (Serovar 6a) wurde mit dem Pathogenitätsfaktoren Listeriolysin und ActA aus Listeria monocytogenes "aufgerüstet". Um die Expression dieser Gene regulieren zu können, wurde als drittes Gen der in trans positiv-wirkende Transkriptionsfaktor (PrfA) in den gentechnisch manipulierten L. innocua Stamm einkloniert. Die Gegenwart von PrfA macht die Expression der Virulengene von der Wachstumstemperatur abhängig. Da dieser rekombinante L.innocua Stamm keine Internaline besitzt, d.h. von sich aus nicht invasiv ist, kann er nicht in die oben erwähnten Zellen (Ptk2; Hep-2) eindringen. Wünscht der Experimentator zusätzlich auch solche Zelle infizieren zu können, so müssen die Bakterien zusätzlich mit den Internalinen InlA und/oder InlB ausgestattet werden. Die Experimente des vorliegenden Beispiels zeigen, daß es zur Aufnahme des L.innocua (hly+; actA+) Stammes durch professionelle Phagozyten dieser bakteriellen Produkte (Internaline) nicht bedarf. Nach ihrer Phagozytose setzt der L.innocua Stamm (hly+; actA+) das Eiweiß Listeriolysin zur Lyse der Phagolysosomen der professionellen Phagozyten ein. Im elektronenmikroskopischen Bild ist zu erkennen, daß der genetisch manipulierte L.innocua (hly+; actA+) Stamm im Zytoplasma der professionellen Phagozyten auftaucht. Der Wildtypstamm L.innocua Serovar 6a hingegen wird im Phagolysosom abgetötet und erscheint nicht im Zellzytoplasma. Die Expression des ActA-Proteins erlaubt dem L.innocua (hly+; actA+) Stamm eine Aktin-Zytoskelett abhängige intrazelluläre Bewegungen, die im EM-Bild der Bewegung der L.monocytogenes Stämme ähnlich sieht. Auf Grund des Fehlens weiterer Gene, wie z.B. des plcB Gens, kann sich der hier erwähnte L.innocua (hly+; actA+) Stamm nicht auf Nachbarzellen ausbreiten. Diese spezifische Änderung in der Infektiosität wurde bereits für rekombinante L.monocytogenes ΔplcB Stämme beschrieben.

Die gezielte Auswahl von Genen, hier hly und actA, und deren Transformation in nicht pathogene Bakterien, überträgt die ausgewählten L.monocytogenes Eigenschaften auf nicht pathogene Bakterien. Die Befreiung der Bakterien aus dem "tödlichen" Phagolysosom ist die Voraussetzung für den Transfer fremder DNA in die infizierten Zellen. Die zur Umprogrammierung der animalen Zelle zu übertragende DNA wird dabei in Konstrukte integriert, wie sie oben bereits für attenuierte L.monocytogenes Bakterien - die bereits als solche erfindungsgemäß eingesetzt werden können - beschrieben wurden. Die Freisetzung der genetischen Information erfolgt erfindungsgemäß durch (i) Schaffung auxogener Mutanten (Deletion endogener, lebensnotwendiger Gene), durch (ii) Einführung von "Suicid-Genen", (iii) durch induzierte Aufnahme in Endosomen und Abtötung darin oder (iv) durch eine zeitlich definierte und auf die Abtötung der Bakterien in einem Zielorgan oder Gewebe bezogene Antibiotikatherapie.

Die Experimente dieses Beispiels zeigen exemplarisch, wie von Hause aus nicht pathogene Bakterien sukzessiv "aufgerüstet" werden können. Durch Ausrüstung mit definierten bakteriellen Faktoren (hier Gen d.h. Eigenschaften natürlich invasiver Bakterien) lassen sich ansonsten für das TGC-Verfahren primär ungeeignete Bakterien, von Experimentator derart manipulieren und steuern, daß sie für eine kontrollierte Infektion und Übertragung von DNA in die animale Zelle (synonym: Gewebe, Organ, Ganztier, Mensch) eingesetzt werden können.

### c) Freisetzung durch exogenen Suizid: Einklonierung von Suizidgenen: (siehe oben, C.4 unter Aufzählungspunkt (b))

Suizid-Gene, die nach dem Eindringen in die Wirtszelle aktiviert werden und zum Absterben der Bakterien führen, können den Bakterien in Form von Lysisgenen aus Bakteriophagen, zum Beispiel mit dem S-Gen des Bakteriophagen Lambda oder Analoga [37], oder mit Killergenen aus Plasmiden [38] zugeführt werden. Diese Gene stehen unter der Kontrolle eines intrazellulär induzierbaren Promotors (zum Beispiel pagC-Promotor aus Salmonella [38]).

### d) Freisetzung durch Antibiotikatherapie: Gezielte Freisetzung von Fremd-DNA in der Lunge nach Tröpfchen-Infektion von Listeria monocytogenes (siehe oben, D.1 unter Aufzählungspunkt (b))

Die Infektion mit den Bakterien erfolgte nach dem Verfahren der "Body plethysmography in spontaneously breathing mice" gemäß R. Vijayaraghavan [Arch. Toxicol. 67: 478-490 (1993)]. Bei dem Versuch wurden die Mäuse einzeln in einer Inhalationskammer für eine halbe Stunde einem Aerosol von einem Milliliter Bakteriensuspension ausgesetzt, das insgesamt 5000 Bakterien enthielt. Diese Anzahl von Bakterien entspricht einer LD50 Dosis bei intraperitonealer Gabe der Bakterien. Um den Verlauf der Infektion in Echtzeit verfolgen zu können, wurden die Bakterien erneut mit einem EGFP-Genkonstrukt transformiert. Durch Fluoreszenzanalyse des im Gewebe gebildeten EGFP-Proteins wurde der Weg der Bakterien im Tiermodell verfolgt. Demnach dringen die Bakterien innerhalb einer halben Stunde in die Säulen- und Endothel-Zellen des Luftweges ein. Zu diesem Zeitpunkt sind keine Bakterien in anderem Gewebe bzw. Organen des infizierten Tiers, wie z.B. Milz, Leber, Gehirn, zu finden. Die Infektion bleibt für bis zu 18 Stunden ausschließlich auf die Lunge begrenzt. Erst nach 24h werden auch andere Organen befallen.

Der Versuch zeigt, daß die Ausbreitung der Bakterien nach Tröpfcheninfektion auf das Primärorgan begrenzt werden kann, wenn in deren Lebensfähigkeit eingegriffen wird. Zwei Wege, dies zu erreichen, sind die Verwendung attenuierter Mutanten (z.B. deletiert im "Ausbreitungsgen" ActA) und/oder die Zerstörung der Bakterien durch Antibiotika-Therapie Zugabe zu einem vom Experimentator vorgegebenen Zeitpunkt, d.h. in einem vom Experimentator vorgegebenen Organ.

### Beispiel 3: Beschreibung der TGC-Vektoren

TGC-Vektoren sind episomale DNA, zum Beispiel Plasmide mit geringer Aufnahmefähigkeit von Fremd-DNA (pMB-Abkömmlinge, die für einzelne Gene ausreicht), oder mit größerer DNA-Aufnahmefähigkeit (wie bei P1- oder F-Plasmiden), um die somatische Transgenität für komplexe Biosynthese-Wege zu schaffen.

In allen Fällen handelt es sich um Plasmide, die in den zur genetischen Veränderung und zur Anzucht für das TGC-Verfahren eingesetzten Bakterien Wirten repliziert werden. Als Beispiel für einen Zwischenwirt, in dem genetische Bausteine konstruiert werden können, eignen sich E.coli oder andere in der rekombinanten DNA-Technologie üblicherweise eingesetzte Bakterien. Als TGC-Sicherheitsstamm eignet sich unter anderem L.monocytogenes oder die anderen obengenannten Bakterien, die als TGC-Amme fungieren. Um diese Bedingung zu erfüllen, enthalten die Plasmide die wirtsspezifischen Plasmid-Replikon-Sequenzen. Bei der Erzeugung der rekombinanten DNA müssen transformierte von "nackten" Wirtszellen unterschieden werden. Als Selektionsprinzipien können dabei die gängigen Antibiotikaresistenzgene eingesetzt werden.

### Beispiel 4: Transformation von L.monocytogenes-Sicherheitsstämmen zu TGC-Ammen

Die Transformation von L.monocytogenes erfolgt nach einem modifizierten Protokoll von Park und Stewart [40].

Dazu werden Bakterien bis zu einer optischen Dichte von OD₆₀₀ = 0,2 angezogen. Dem Anzuchtsmedium wird Ampicillin (10µg/ml) und 1 mM Glyzin zugegeben. Es erfolgt dann eine weitere Vermehrung bis zu einer OD₆₀₀ von 0,8 bis 1,0. Die Zellen werden durch Abzentrifugation geerntet und in 1/250 Vol. kaltem Elektroporationspuffer (1 mM Hepes, pH 7,10; 0,5 M Sucrose) aufgenommen. Als Vorbereitung zur Elektroporation werden die Bakterien bis zu viermal gewaschen.

Zur Elektroporation werden 50 µl der vorbereiteten Zellen in eine Elektroporationsküvette gegeben, die Elektroporation erfolgt bei 10 kV/cm, 400 Ohm, 25 µF unter Einsatz von ca 1 µg DNA.

Danach kommen die Zellen sofort auf Eis und werden in 10-fachem BHI-Medium aufgenommen und für 2 Stunden bei 37°C unter vorsichtigem Schütteln bebrütet. Nach dieser Zeit werden die Zellen ausplattiert und bei der gewünschten Temperatur bebrütet. Die Effizienz der Transformation nach dieser Methode beträgt 10⁴ bis 10⁵ Transformaten pro µg eingesetzter Plasmid DNA.

### Beispiel 5: Beschreibung der Anzucht von TGC-Ammen zum Einsatz im TGC-Verfahren

Listerien wurden bevorzugt in der Brain-Heart-Infusion Broth zum Beispiel BHI der Firma Difco angezüchtet. Alternativ und für spezielle Anwendungen (radioaktive Markierung listerieller Proteine) können die Bakterien in Tryptic Soy broth (TSB) oder in Listerien Minimal Medium (LMM) gezüchtet werden [36]. Die Bakterien werden abzentrifugiert und mehrfach im geeigneten Transfer-Medium, zum Beispiel einem Bicarbonat-haltigen Puffer, gewaschen.

Derart vorbereitete Bakterien können unter Zusatz von 15% Glycerin Lösung bei -80°C für mindestents 6 Monate aufbewahrt werden, bevor sie in das TGC-Verfahren eingesetzt werden.

### Beispiel 6: TGC-Verfahren - Verfüttern der TGC-Ammen

Zur Einleitung des TGC-Verfahrens wird den Tieren für einige Stunden das Trinkwasser entzogen. Die (TGC-Ammen: TGC-DNA im gewünschten TGC-Stamm) werden in einem Bicarbonat-haltigen Puffer geeigneter Konzentration aufgenommen und den Tieren oral, durch Inhalation oder per Injektion (parenteral, intramuskulär, intraperitoneal oder direkt in das gewünschte Zielorgan) verabreicht. Die Art der Applikation richtete sich nach dem physiologischen Infektionsweg der entsprechenden TGC-Amme. Die Auswahl des Keimes der als TGC-Sicherheitsstamm eingesetzt wird, richtet sich nach Zielorgan und wird entsprechend des Infektionsweges und entsprechend des Organotropismus des jeweiligen Bakteriums festgesetzt. Die Dosis an Bakterien wird so gewählt, daß die gewünschte organotrope Einschleusung der TGC-Amme erzielt wird. Die Menge und die Art der Applikation der Bakterien richtet sich dabei nach dem jeweiligen Bakterium, ist aber auch vom Wirt und dem Zielorgan abhängig (siehe auch Beipiel 2).

### Beispiel 7: Durchführung der somatischen Gentherapie

Als Beispiele für eine somatische Gentherapie sind hier aufgeführt:
- Die Therapie der cystischen Fibrose (CF): Dazu muß der Keim dem zu therapierenden Patienten durch Inhalation verabreicht werden. Bei dem Bakterium handelt es sich vorzugsweise um einen Keim, der über Tröpfcheninfektion übertragen wird. In dem Bakterium befindet sich das CFTR-Gen, das den bei der CF maßgeblichen Defekt kurieren kann. Das Bakterium dringt in die Säulenzellen des Luftweges (airway lumen-facing columnar cells) ein und transfiziert diese mit der CFTR-DNA integriert in den TGC-Vektor. Die Zellen werden somatisch transgen, der Defekt wird kuriert.
- Durch somatische Gentherapie mit dem humanen β-Globulingens kann die β-Thalasaemie behandelt werden. Dazu werden ex vivo Stammzellen der haematopoetischen Reihe mit einem TGC-Sicherheitsstamm infiziert, der das β-Globulingen auf die Stammzelle überträgt. Durch Behandlung der Zellen in der Zellkultur wird das infizierende Bakterium eliminiert und die transgene Zelle für die Rückübertragung auf den Menschen vorbereitet. Diese Übertragung erfolgt durch intravenöse Gabe.
- Bei der Therapie des Hurler Syndroms werden primitive CD34-positive Zellen des Knochenmarks mit dem α-L-Iduranidase-Gen transfiziert. Der Weg der Gentherapie und der Rückübertragung der Zellen auf den Patienten entspricht dem im vorherigen Punkt.
- Bei der Gentherapie der Fanconi Anaemie wird das Gen der Fanconi Anaemie Komplementationsgruppe C (FACC) zur somatischen Gentherapie eingesetzt. Zielzellen der Infektion mit der TGC-Amme sind hierbei erneut CD34-positive Zellen des Knochenmarks.

### Beispiel 8: Kontrolle für den Erfolg der induzierten somatischen Transgenität

Nach dem Transfer der TGC-DNA in den TGC-Wirt ist der Erfolg des TGC-Verfahrens nachzuweisen. Dazu eignen sich immunologische Nachweise des Genproduktes (Proteins) durch Immunoassays wie den ELISA, den Immunoblot oder andere bekannte Nachweise, die auf einer Antigen-Antikörperreaktion beruhen. T-Zell-Antworten können in speziellen Assays abgerufen werden und werden immer dann angewendet, wenn es sich bei dem Antigen um eine Substanz handelt, die über MHC-Klasse I vermittelte Immunantworten erkannt wird.

Handelt es sich bei dem produzierten Protein um ein Enzym, so kann dessen biologische Aktivität in Form der enzymatischen Aktivitätstestung erfolgen. Besitzt das Protein zusätzlich eine biologische Aktivität, so wird die Leistungsfähigkeit des gebildeten Proteins durch biologische Assays abgerufen.

Für Proteine, die eine passive oder aktive Immunisierung des TGC-Wirtes induzieren, wird die Protektion gegenüber dem auslösenden Agens getestet. Es kann sich zum Beispiel um die Verhinderung der Besiedlung, der Infektion (oder der apparenten Erkrankung) des Versuchstieres nach Belastung mit dem pathogenen Organismus (Bakterium oder Virus) handeln.

### Beispiel 9: Ernte des Proteins

Die Gewinnung des produzierten Proteins erfolgt durch Techniken, die jeder in der Landwirtschaft tätigen Person bekannt sind:
- ist der TGC-Wirt eine Kuh oder ein anderes laktierendes Nutztier und das Euter das infizierte Organ, so werden die bekannten Techniken des Melkens eingesetzt;
- wenn Geflügeltiere wie Hühner als TGC-Wirt eingesetzt wurden, so werden die Eier gesammelt und der Protein-Reinigung zugeführt;
- die Aufarbeitung von Proteinen aus Organen, deren Produkte nicht nach außen abgegeben werden, erfolgt durch die Gewinnung des entsprechenden Organs, wozu in der Regel eine Schlachtung nötig sein wird, zum Beispiel bei Fischen;
- ist das Blut das somatisch transgene Gewebe, so wird das gewünschte Produkt nach Venenpunktion aus dem Blut oder seinen Zellen gewonnen und mit dem Fachmann bekannten Methoden aufgereinigt.

### Beispiel 10: Anreinigung des Proteins

Eine Vorreinigung des zu produzierenden Proteins erfolgt durch Trennungsverfahren, die sich primär physikalischer oder physikochemischer, dem Fachmann wohl bekannter Methoden bedienen. Dazu gehören Fällungen der Proteine mit Salzen (zum Beispiel Ammoniumsulfat), mit Säuren (zum Beispiel Trichloressigsäure) oder unter Einwirkung von Hitze oder Kälte.

Eine grobe Trennung wird auch mittels der Säulenchromatographie erreicht. Alle hier eingesetzten Verfahren richten sich sehr stark nach den primären Medien, in denen sich das jeweilige Protein anreichert. So sind zum Beispiel für die Aufarbeitung von Milch oder Eiern in der Industrie viele Methoden bekannt, die auch auf die hier geschilderte Erfindung anwendbar sind. Gleiches gilt für die Aufarbeitung von Blut als somatisch-transgenes Gewebe. Hier kann auf die Erfahrungen auf die Transfusionsmedizin, spezielle auf die Aufarbeitung und Reinigung von Blutgerinnungsfaktoren zurückgegriffen werden.

### Beispiel 11: Reinigung des Proteins

Zur endgültigen Reinigung der Proteine sind alle Methoden einsetzbar, die für konventionelle Reinigungen von Proteinen Anwendung finden. Dazu gehören
- die Reinigung mittels Affinitätschromatographie zum Beispiel unter Ausnutzung der Rezeptor-Ligand Interaktion;
- die Darstellung von Fusionsproteinen mit sog. "Tags", die zur spezifischen Interaktion mit einer Matrix der Chromatographie eingesetzt werden können (zum Beispiel Poly-Histidin-Tag und Nickel-Säulen-Chromatographie; die Streptavidin-Biotin Technologie der Affinitätsreinigung). Die Tags können bei fachgerechter Einführung einer entsprechenden Proteaseschnittstelle durch späteren Proteasen-Verdau wieder entfernt werden;
- die Reinigung mittels spezifischer Antikörper (Immunaffinitätschromatographie);
- die Ausnutzung natürlicher Affinitäten zwischen dem Zielprotein und anderen Proteinen, Kohlehydraten oder anderen Bindungspartnern wie im Fall des Toxin A von Clostridium difficile, dessen Bindungsfähigkeit an Thyroglobin bei 4°C und dessen anschließender Elution durch Temperaturerhöhung auf 37°C.

### Beispiel 12: Produktion von TGC-Proteinen:

Die Liste der durch das TGC-Verfahren herstellbaren Proteine ist theoretisch unbegrenzt und umfaßt vor allem den Bereich der Hormone, Regulationsfaktoren, Enzyme, Enzyminhibitoren, humane monoklonale Antikörper sowie die Herstellung von Oberflächenproteinen pathogener Mikroorganismen oder viraler Hüllproteine zur ungefährlichen Herstellung diagnostischer Tests und verträglicher Impfstoffe. Es handelt sich dabei sowohl um Massenartikel wie humanes Serumalbumin als auch um Proteine, die in geringen Mengen eingesetzt werden wie Hirudin, Blutgerinnungsfaktoren, Antigene zur Tumorprophylaxe und zur aktiven Immunisierung (zum Beispiel Papilloma-Antigen) sowie um Antikörper zur passiven Immunisierung.

### Literaturverzeichnis:

1. Cossart P. und B.B. Finlay "Exploitation of mammalian host cell functions by bacterial pathogens Science 276: 718-725.
2. Falkow, S., Isberg, R.R. und Portnoy D.A. (1992) The interaction of bacteria with mammalian cells Ann. Rev. Cell Biol. 8:333-63.
3. Weinberg, A.N. Zoonoses S. 291-2795; In Principle and Practice of Infectious Diseases, Eds. Mandell, Douglas und Bennett, J.E. und Dolin R. Churchill Linvingstone New York, 1995.
4. Farber J.M. und Peterkin, P.J. (1991) Listeria monocytogenes - a foodborne pathogen Microbiol. Rev. 55: 476-511.
5. Thoen C.O. (1994) Tuberculosis in wild and domestic animals pp. 157-62, in Tuberculosis: Pathogenesis, Protection and Control ; ASM, Washington DC 20005.
6. von Hase, U., Pulz, M., Windorfer, A: EHEC in Niedersachsen, Januar 1995 - August 1997. Niedersächs. Ärtzebl (1997) 20-23 u 38-40.
7. Swaminathan B., Rocourt J., und Bille J.. (1995) Listeria. In: Manual of Clinical Microbiology. Eds: Murray, P.R., Baron E.J., Pfaller M.A., Tenover F.C., und Yolken R.H. S.341-348. ASM-Press Washington DC.
8. Hof, H., Nichterlein, T. und Kretschmar, M. (1997) Management of Listeriosis. Clin. Microbiol. Rev. 10: 345-357. 8a. Simon, C. und Stille, W. Antibiotikatherapie, Schattauerverlag,
9. Chakraborty, T. und Wehland, J (1997) The host cell infected with Listeria monocytogenes pp. 271-290; in Host response to intracellular pathogens, Ed. S.H.E. Kaufmann R.G. Landes co., Austin, USA.
10. Gaillard, J.L., Berche, P., Frehel, C., Gouin, E. und Cossart, P. (1991) Entry of L. monocytogenes is mediated by internalin, a repeat protein reminiscent of surface antigens from gram-positive cocci Cell: 65; 1127-1141.
11. Lingnau, A., Domann, E., Hudel, M., Bock, M., Nichterlein, T., Wehland, J. und T. Chakraborty (1995) Expression of inlA and inlB in L. monocytogenes EGD, whose products mediate bacterial entry into tissue culture cell lines, by PrfA-dependent and - independent mechanisms Infect. Immun. 63: 3896-3903.
12. Alvarez-Dominguez, C., Carasco-Martin, E. und Levya-Cobian F (1993) Role of complement component Clq in phagocytosis of L. monocytogenes by murine macrophage-like cell lines . Infect. Immun. 61: 3664-3672.
13. Dunne, D.W., Resnick, D., Greenberg, J., Krieger, M. und Joiner, K.A. (1994) The type I macrophage scavenger receptor binds to Gram-positive bacteria and recognizes lipoteichcoic acid. Proc. Natl. Acad. Sci. USA 91: 1863-7.
14. Gaillard, J-L, Berche, P. und Sansonetti, PJ (1986) Transposon mutagenesis as a tool to study the role of hemolysin in virulence of Listeria monocytogenes Infect. Immun. 52: 50-55.
15. Theriot, J.A., Rosenblatt, J., Portnoy, D.A., Goldschmidt-Clermont, P.J. und Mitchison, T.J. (1994) Involvement of profilin in the actin-based motility of Listeria monocytogenes in cells and cell-free extracts. Cell 76: 505-517.
16. Chakraborty, T., Ebel, F., Domann, E., Niebuhr, K., Gerstel, B., Pistor, S., Temm-Grove, CJ, Jockusch, BM., Reinhard, M., Walter, U. und Wehland, J. (1995) A focal adhesion factor directly linking intracellularly motile Listeria monocytogenes and Listeria ivanovii to the actin-based cytoskeleton of mammalian cells EMBO J. 14: 1314-21.
17. Vazquez-Boland, J.A., Kocks, C., Dramsi, S., Ohayon, H., Goeffroy, C., Mengaud, J. und Cossart, P. (1992) Nucleotide sequence of the lecithinase operon of L. monocytogenes and possible role of lecithinase in cell-to-cell spread. Infect. Immun. 60: 219-30.
18. L'Hopital, S.J., Marly, J., Pardon, P. und Berche, P. (1993) Kinetics of antibody production against listeriolysin O in sheep with listeriosis. J Clin. Microbiol. 31: 1537-40.
19. Domann, E., Zechel, S., Lingnau, A., Hain, T., Darji, A., Nichterlein, T., Wehland, J. und Chakraborty, T. (1997) Identification and characterization of a novel PrfA-regulated gene in Listeria monocytogenes whose product, IrpA, is highly homologous to internalin proteins, which contain leucine-rich repeats. Infect. Immun. 65: 101-9.
20. Grenningloh, R., Darji, A., Wehland, J., Chakraborty, T. und Weiss, S. (1997). Listeriolysin and IrpA are major protein targets of the human humoral response against Listeria monocytogenes. Infect. Immun. 65: 3976-3980, 1997.
21. Shen, H., Slifka, M.K., Matloubian, M., Jensen, E.R., Ahmed, R. und Miller, J.F. (1995) Recombinant Listeria monocytogenes as a live vaccine vehicle for the induction of protective anti-viral cell-mediated immunity. Proc. Natl. Acad. Sci. USA. 92: 3987-91.
22. Slifka M.K., Shen, H., Matloubian, M., Jensen, E. R., Miller, J.F. und Ahmed, R. (1996) Antiviral cytotoxic T-cell memory by vaccination with recombinant Listeria monocytogenes. J. Virology 70: 2902-10.
23. Jensen, E.R., Selvakumar, R., Shen, H., Ahmed, R., Wettstein, F.O. und Miller J.F. (1997) Recombinant Listeria monocytogenes Vaccination Eliminates Papillomavirus-Induced Tumors and Prevents Papilloma Formation from Viral DNA. J. Virol. 71: 8467-8474.
24. Schafer, R., Portnoy, D.A., Brassell, S.A. und Paterson, Y. (1992). Induction of a cellular immune response to a foreign antigen by a recombinant Listeria monocytogenes vaccine. J. Immunol. 149: 53-9.
25. Ikonomidis, G., Paterson, Y., Kos, F.J. und Portnoy, D.A. (1994). Delivery of a viral antigen to the class I processing and presentation pathway by Listeria monocytogenes. J. Exp. Med. 180: 2209-18.
26. Frankel, F.R., Hegde S., Lieberman, J. und Paterso, Y. (1995) Induction of cell-mediated immune responses to human immunodeficiency virus type 1 Gag protein by using Listeria monocytogenes as a live vaccine vector. J. Immunol. 155: 4775-82.
27. Pan, Z.K., Ikonomidis, G., Lazenby, A., Pardoll, D. und Paterson, Y. (1995) A recombinant Listeria monocytogenes vaccine expressing a model tumour antigen protects mice against lethal tumour cell challenge and causes regression of established tumours. Nature Med. 1: 471-7.
28. Dramsi, S., Kocks, C., Forestier, C. und Cossart, P. (1993) Internalin-mediated invasion of epithelial cells by L. monocytogenes is regulated by bacterial growth, temperature and the pleiotropic activato, prfA. Mol. Microbiol. 9: 931-41.
29. Alvarez-Dominguez, C., Vazquez-Boland, J.A., Carrasco-Marin, E., Lopez-Mato, P. und Leyva-Cobian F. (1997). Host cell heparan sulfate proteoglycans mediate attachment and entry of Listeria monocytogenes, and the listerial surface protein ActA is involved in heparan sulfate receptor recognition. Infect. Immun. 65: 78-88.
30. Domann, E., Wehland, J., Rohde, M., Pistor, S., Hartl, M., Goebel, W., Leimeister-Wächter, M., Wuenscher, M. und Chakraborty, T. (1992) A novel bacterial virulence gene in L. monocytogenes required for host microfilament interaction with homology to the proline rich region of vinculin. EMBO. J. 11: 1981-1990.
31. Kocks, C., Gouin, E., Tabouret, M., Berche, P., Ohayon, M. und Cossart, P. (1992) Listeria monocytogenes induced actin assembly requires the actA gene product, a surface protein. Cell 68: 521-31.
32. Armstrong, D. Listeria monocytogenes (1995) In Principle and Practice of Infectious Diseases, Eds. Mandell, Douglas und Bennett, J.E. und Dolin R.. S.1880-1885. Churchill Linvingstone New York, 1995.
32a. Boucher, R.C. (1996). Current status of CF gene therapy. Trends in Genetics 12: 81-84.
32b. Bank A. (1996). Human somatic cell gene therapy. BioEssays 18: 999-1007.
33. O'Callaghan D, Maskell D, Tite J, Dougan G (1990). Immune responses in BALB/c mice following immunization with aromatic compound or purine-dependent Salmonella typhimurium strains. Immunology 69:184-189.
34. Tacket, C.O., Sztein, M.B., Losonsky, G.A., Wasserman, S:S., Nataro, J.P., Edelman, R., Pickard, D., Dougan, G., Chatfield, S., und Levine, M.M. (1997). Safety of Live Oral Salmonella typhi Vaccine Strains with deletions in htrA and aroC, aroD and immune response in humans. Infect. Immun. 65: 452-456.
35. Curtiss III, R. (1989) Attenuated Salmonella strains as live vectors for the expression of foreign antigens. In New generation vaccines: The molecular approach (ed. M.M. Levine und G. Woodrow) p. 161. Marcel Dekker, New York.
36. Hopkins, S., Kraehenbuhl, J.-P., Schödel, F., Potts, A, Peterson, D., De Grandi, P., und Nardeli-Haefliger, D. (1995). A recombinant Salmonella typhimurium vaccine induces local immunity by four different routes of immunization. Infect. Immun. 63: 3279-3286.
37. Berkmen, M., Benedik, MJ., und Blasi, U. (1997). The Serratia marcescens NucE protein functions as a holin in Escherichia coli. J. Bacteriol. 179: 6522-6524.
38. Diaz, E., Munthali, M., de Lorenzo, V., und Timmis KN (1994). Universal barrier to lateral spread of specific genes among microorganisms. Mol. Microbiol. 13: 855-861.
39. Hohmann, El., Oletta, CA., Loomis, WP, und Miller, SI (1995). Macrophage-inducible expression of a model antigen in Salmonella typhimurium enhances immunogenicity. Proc. Natl. Acad. Sci. (USA) 92:2904-2908.
40. Park S.F. und Stewart G.S. (1990). High-efficiency transformation of Listeria monocytogenes by electroporation of penicillin-treated cells. Gene 94:129-132.
41. Premaratne, R.J., Lin, W.J. und Johnson E.A. (1991) Development of an improved chemically defined minimal medium for L. monocytogenes. Appl. Environ. Microbiol. 57: 3046-48.

### SEQUENZPROTOKOLL

### ALLGEMEINE ANGABEN

- ANMELDER:: 1. Privatdozent
   Dr. Christoph von Eichel-Streiber

   Bingerweg 15
   55444 Schweppenhausen
   Bundesrepublik Deutschland
   Tel.: 06724/33 98
   Fax : 06724/33 98
2. Prof. Dr. Trinad Chakraborty
   Seltersweg 85
   35390 Gießen
   Bundesrepublik Deutschland
   Tel.: 0641/76 536
   Fax : 0641/99 41 259
- BEZEICHNUNG DER ERFINDUNG:: TGC-Verfahren zur Induktion einer zielgerichteten, somatischen Transgenität
- ANZAHL DER SEQUENZEN:: 2
- ZUSTELLANSCHRIFT :: Patentanwälte
Dr. Rainer A. Keil
Ludwig R. Schaafhausen
Nanno M. Lenz
Dr. K.-H. Meyer-Dulheuer
Eysseneckstraße 31
D-60322 Frankfurt am Main

### COMPUTERLESBARE FASSUNG

- DATENTRÄGER :: Diskette
- COMPUTER :: IBM PC compatible
- OPERATING SYSTEM:: PC-DOS/MS-DOS
- SOFTWARE :: Word Perfect 6.0

### Angaben zur Sequenz ID-No 1:

- Länge :: 1260 Basenpaare
- Art :: Nukleinsäure und davon abgeleitete Aminosäuresequenzen
- Strangform :: Einzelstrang
- Topologie :: linear
- Herkunft :: Listeria monocytogenes Stamm EGD Serotyp 1/2a
- Merkmal :: Sequenz des dapE Gens, das eines der für die Synthese der Diaminopimelinsäure notwendigen Schlüsselenzyme ist. Die Aminosäure sequenz ist hoch homolog zu N-succinyl-L-diaminopimelic acid desuccinylase (dapE) unter anderem aus Escherichia coli, Bacillus subtilis, Lactobacillus spp., Mycobacterium tuberculosis.

- Aminosäuresequenz:: 318 Aminosäuren
- Nukleotidsequenz :: 1260 Nukleotide

1 TGCCTTTATA GAGAACGGGA AAACATAGAG TGGAATTCAT AGAAAGAGGG
51 CGTGAAATAT GGACCAACAA AAAAAGATTC AAATTTTAAA GGACTTGGTA
101 AATATTGATT CGACTAATGG GCATGAAGAA CAAGTTGCGA ACTATTTGCA
151 AAAGTTGTTA GCTGAACATG GTATTGAGTC CGAAAAGGTA CAATACGACC

201 TAGACAGAGC TAGCCTAGTA AGCGAAATTG GTTCCAGTAA CGA GAA GGT T
R E G
251 TG GCA TTT TCA GGG CAT ATG GAT GTA GTT GAT GCG GGT GAT GTA TCT AAG
L A F S G H M D V V D A G D V S K -
301 TGG AAG TTC CCA CCT TTT GAA GCG ACA GAG CAT GAA GGG AAA CTA TAC GG
W K F P P F E A T E H E G K L Y G -
351 A CGC GGC GCA ACG GAT ATG AAG TCA GGT CTA GCG GCG ATG GTT ATT GCA A
R G A T D M K S G L A A M V I A -
401 TG ATT GAA CTT CAT GAA GAA AAA CAA AAA CTA AAC GGC AAG ATC AGA TTA
M I E L H E E K Q K L N G K I R L -
451 TTA GCA ACA GTT GGG GAA GAG ATC GGT GAA CTT GGA GCA GAA CAA CTA AC
L A T V G E E I G E L G A E Q L T -
501 A CAA AAA GGT TAC GCA GAT GAT TTA CAT GGT TTA ATC ATC GGC GAA CCG A
Q K G Y A D D L H G L I I G E P -
551 GT GGA CAC AGA ATC GTT TAT GCG CAT AAA GGT TCC ATT AAT TAT CCC GTT
S G H R I V Y A H K G S I N Y P V -
601 AAA TCC ACT GGT AAA AAT GCC CAT AGT TCG ATG CCG GAA TCT GGT GTG AA
K S T G K N A H S S M P E S G V N -
651 T GCG ATT GAT AAC TTG CTG CTA TTT TAT AAT GAA GTA GAA AAA TTC GTG A
A I D N L L L F Y N E V E K F V -
701 AA TCA GTT GAT GCT ACT AAC GAA ATA TTA GGC GAT TTT ATT CAT AAT GTC
K S V D A T N E I L G D F I H N V
751 ACC GTA ATT GAT GGT GGA AAT CAA GTC AAT AGT ATC CCT GAA AAA GCA CA
T V I D G G N Q V N S I P E K A Q -
801 A CTG CAA GGG AAT ATT CGC TCG ATT CCA GAA ATG GAT AAT GAA ACA GTG A
L Q G N I R S I P E M D N E T V -
851 AA CAA GTG CTA GTG AAG ATT ATC AAT AAG TTA AAC AAA CAG GAA AAT GTG
K Q V L V K I I N K L N K Q E N V -
901 AAT CTG GAA TTA ATA TTT GAT TAT GAT AAA CAA CCA GTA TTT AGT GAT AA
N L E L I F D Y D K Q P V F S D K -
951 A AAT TCG GAT TTA GTC CAC ATT GCT AAG AGC GTA GCA AGC GAC ATT GTC
N S D L V H I A K S V A S D I V
1001 AAA GAA GAA ATC CCA TTA CTC GGT ATT TCC GGA ACA ACC GAT GCA GCA GA
K E E I P L L G I S G T T D A A E -
1051 A TTT ACC AAA GCT AAG AAA GAG TTC CCA GTG ATT ATT TTT GGA CCA GGA A
F T K A K K E F P V I I F G P G -
1101 AC GAA ACC CCT CAC CAA GTA AAC GAA AAT GTT TCT ATA GGA AAT TAT TTG
N E T P H Q V N E N V S I G N Y L -
1151 GAG ATG GTA GAT GTT TAC AAA CGG ATT GCC ACC GAG TTT TTA TCT TGA TGA
E M V D V Y K R I A T E F L S STOP

1201 AACTTTAACT TTACTTATTT CCCGATATAA AATAAGTAAT TAATAGAAGT
1251 CTAGTATTTG 1260

### Angaben zur Sequenz ID-No 2:

- Länge :: 1337 Basenpaare
- Art :: Nukleinsäure und davon abgeleitete Aminosäuresequenzen
- Strangform :: Einzelstrang
- Topologie :: linear
- Herkunft :: Listeria monocytogenes Stamm EGD 1/2a
- Merkmal :: Sequenz des "cold shock proteins" cspL; dieses Protein ist für die Lebensfähigkeit der Listerien bei niedrigen Temperaturen essentiell.

- Aminosäuresequenz:: 66 Aminosäuren
- Nukleotidsequenz :: 1337 Nukleotide

1 GAGGCAAGTG GACTAATCAT AAAGTTTTTG GCGATGCAAC TGCGATTTTG
51 GCAGGAGATG CTTTACTAAC GCTCGCTTTT TCTATTTTAG CTGAAGACGA
101 TAATTTATCT TTTGAGACAC GCATTGCTTT GATTAACCAA ATTAGTTTTA
151 GTAGCGGTGC AGAAGGAATG GTTGGTGGTC AACTTGCAGA CTTGGAAGCG
201 GAAAACAAAC AAGTGACGCT AGAAGAGTTA TCATCCATTC ATGCACGAAA
251 AACGGGTGAA TTATTAATTT ATGCTGTAAC CTCTGCAGCA AAAATTGCGG
301 AAGCTGATCC AGAACAAACG AAACGCTTAC GAATTTTTGC AGAGAATATT
351 GGGATTGGAT TTCAAATTAG CGACGATATT TTAGATGTAA TTGGTGATGA
401 AACGAAAATG GGTAAAAAGA CAGGGGCCGA CGCTTTTCTG AATAAAAGTA
451 CCTATCCCGG ATTACTCACG CTTGATGGGG CAAAAAGGGC ATTAAATGAG
501 CATGTTACGA TTGCAAAGTC AGCGCTTTCA GGGCATGATT TCGATGATGA
551 AATTCTCTTG AAACTTGCTG ATTTAATCGC ACTTAGAGAA AATTAATCAT
601 AATTATCTAG TAATTTCAAA ATTTTTTCAC ATATATAATT CAAATTGATT
651 TGCTTTTCCT AAAATACCGT GTTATACTAA TGTAAGATTA TTTTTGTGGG
701 TGAAAGATAC GATTGTGAAC AACTTTCCAT CTCGTGCCGT TAAGCAAGAA

751 TAGTAAATAA TTAGTGTGCA TAACACACGA GGAGGAACAT GAAC ATG GAA
M E
801 CAA GGT ACA GTA AAA TGG TTT AAC GCA GAA AAA GGA TTT GGT TTT ATC GA
Q G T V K W F N A E K G F G F I E
851 A CGC GAA AAC GGT GAC GAT GTA TTC GTA CAT TTC AGC GCT ATC CAA GGC G
R E N G D D V F V H F S A I Q G
901 AC GGA TTC AAA TCT TTA GAC GAA GGT CAA GCA GTA ACT TTC GAC GTT GAA
D G F K S L D E G Q A V T F D V E
951 GAA GGC CAA CGC GGA CCT CAA GCA GCT AAC GTT CAA AAA GCG TAA TTC TA
E G Q R G P Q A A N V Q K A STOP

1001 TTTTTTGAAT AAGAAAAAGC AAATCATTTC GGTGATTTGC TTTTTTATTT
1051 GTCTAAAATT ATTTTACCTT GTTTGGTTTA ATGGCGATTG TTTGCTATAA
1101 TAAGAACAAT TAATCGAGAA AAAAGACCTT GCACGCATTC ATGCGAGTGG
1151 CTCTTTGGAA AGTGAGTTGT TTTTATTTGG ATCTTTTAAA GATAAAGGAT
1201 CCTTCCTTTA TGAAGCGATT GGATATACAA GAATTAGAAG CACTTGCAGC
1251 GGATATTCGC GCTTTTTTAA TTACTTCTAC ATCTAAATCA GGTGGGCATA
1301 TTGGTCCGAA TCTTGGTGTG GTAGAACTAA CGATTGC

## Patentansprüche

1. Bakterien zur Verwendung als Vehikel zum Gentransport und Gentransfer in eukaryonten Zellen eines Organismus zur Induktion einer zielgerichteten somatischen Transgenität in Zellen, Geweben oder Organen ausgenommen der Keimbahn des Organismus, wobei die Bakterien eine in einen episomalen Vektor integrierte Fremd-DNA aufweisen, deren Transkription und Expression unter der Kontrolle eukaryonter, regulatorischer Elemente steht, **dadurch gekennzeichnet,**
- **daß** die Bakterien vital und in dem Organismus lebensfähig sind
- **daß** sie entweder (a) voll pathogen sind,
oder (b) allenfalls derart attenuiert sind, daß eine Verhinderung der Apoptoseinduktion der eukaryonten Zelle und/oder eine Einschränkung der intrazellulären Motilität der Bakterien und/oder eine effiziente Eliminierung der Bakterien nach erfolgtem Gentransfer gewährleistet ist,
oder (c) von Haus aus nicht pathogen und mit zusätzlichen Pathogenitätsfaktoren ausgestattet sind, die die Bakterien zu einer kontrollierten Infektion, zum Vordringen in Organe und Gewebe des Organismus und zur Übertragung von DNA in abgelegene Körperzellen befähigen, und
- **daß** sie im Organismus ihrem typischen Infektionszyklus entsprechend und auf dem für sie typischen Infektionsweg zum Zielorgan und Zielgewebe vordringen und zur Übertragung der Fremd-DNA in abgelegene Körperzellen geeignet sind,
- **daß** der Infektionsweg natürlicherweise oder infolge gezielter genetischer Veränderungen gerichtet und örtlich begrenzt ist, wobei die gezielten genetischen Veränderungen insbesondere Gene betreffen, die die Vermehrung der Bakterien in den eukaryonten Zellen beeinflussen und/oder die Pathogenität der Bakterien im Organismus mindern und/oder das Überleben der Bakterien in der Umwelt nach einer Ausscheidung aus dem Organismus verhindern,
- und **daß** der Infektionszyklus durch den Einsatz von Antibiotika zeitlich begrenzbar und beendbar ist.

2. Bakterien nach Anspruch 1, **dadurch gekennzeichnet,**
**daß** Transkription und Expression der Fremd-DNA unter der Kontrolle von Promotoren und anderen regulatorisch wirkenden Sequenzen stehen , die aus dem vorher ausgewählten Zielorgan des TGC-Verfahrens stammen oder für das Zielorgan optimiert sind.

3. Bakterien nach Anspruch 1 oder 2, **dadurch gekennzeichnet,**
**daß** sie mit einem zusätzlichen exogenen Suizidgen ausgestattet sind.

4. Bakterien nach einem der Ansprüche 1 bis 3, **dadurch gekennzeichnet,**
**daß** es Bakterien der Gattungen Aeromonas, Bartonella, Brucella, Campylobacter, Clostridia, Enterobacteriaceae, Legionella, Listeria, Mycobacterium, Renibacterium, Rhodococcus oder Bakterien aus mit ihnen genetisch oder biochemisch verwandten Gattungen sind.

5. Bakterien nach einem der Ansprüche 1 bis 4, **dadurch gekennzeichnet,**
**daß** sie ein dapE-Gen mit der im Sequenzprotokoll SEQ ID NO. 1 dargestellten Nukleotidsequenz oder ein damit in mindestens 35% der Nukleotidpositionen übereinstimmendes Gen aufweisen, welches deletiert oder durch Blockade oder Mutation inhibiert ist.

6. Bakterien nach Anspruch 5, **dadurch gekennzeichnet,**
**daß** es Bakterien des Stammes Listeria monocytogenes sind.

7. Bakterien nach einem der Ansprüche 1 bis 5, **dadurch gekennzeichnet,**
**daß** sie ein cspL-Gen mit der im Sequenzprotokoll SEQ ID NO. 2 dargestellten Nukleotidsequenz oder ein damit in mindestens 35% der Nukleotidpositionen übereinstimmendes Gen aufweisen, welches deletiert oder durch Blockade oder Mutation inhibiert ist.

8. Bakterien nach Anspruch 7, **dadurch gekennzeichnet,**
**daß** es Bakterien der Gattung Listeria sind.

9. Bakterienstamm Listeria monocytogenes EGD HlyD_{491A}
der bei der DSMZ unter der Nummer 11881 hinterlegt und zur Verwendung gemäß Anspruch 1 geeignet ist.

10. Bakterienstamm Listeria monocytogenes EGD Delta actA Delta plcB
der bei der DSMZ unter der Nummer 11882 hinterlegt und zur Verwendung gemäß Anspruch 1 geeignet ist.

11. Bakterienstamm Listeria monocytogenes EGD Delta cspL 1
der bei der DSMZ unter der Nummer 11883 hinterlegt und zur Verwendung gemäß Anspruch 1 geeignet ist.

12. Bakterien nach einem der Ansprüche 1 bis 11, **dadurch gekennzeichnet,**
**daß** sie das Euter von Kühen oder anderen laktierenden Nutztieren infizieren.

13. Bakterien nach einem der Ansprüche 1 bis 11, **dadurch gekennzeichnet,**
**daß** sie Zellen des Blutes als somatisches Gewebe infizieren.

14. Verwendung eines Bakteriums nach einem der Ansprüche 1 bis 13 zur Erzeugung von Proteinen

15. Verfahren zur Gewinnung von Proteinen in einem tierischen Organismus, ausgenommen der menschlichen Organismus, bei dem Bakterien, die eine in einen episomalen Vektor integrierte Fremd-DNA aufweisen, deren Transkription und Expression unter der Kontrolle eukaryonter, regulatorischer Elemente steht, als Vehikel für einen Gentransport und Gentransfer in eukaryonten Zellen, Geweben oder Organen ausgenommen der Keimbahn des Organismus zur Induktion einer zielgerichteten somatischen Transgenität eingesetzt werden und in den infizierten und transgenen Zellen die Bildung des Fremd-Proteins initiieren, das anschließend aus der Zelle, dem Gewebe oder dem Organ isoliert und gereinigt wird, **dadurch gekennzeichnet,**
- **daß** die Bakterien vital und in dem Organismus lebensfähig sind,
- **daß** sie entweder (a) voll pathogen sind, oder (b) allenfalls derart attenuiert sind , daß eine Verhinderung der Apoptoseinduktion der eukaryonten Zelle und/oder eine Einschränkung der intrazellulären Motilität der Bakterien und/oder eine effiziente Eliminierung der Bakterien nach erfolgtem Gentransfer gewährleistet ist, oder (c) von Haus aus nicht pathogen und mit zusätzlichen Pathogenitätsfaktoren ausgestattet sind, die die Bakterien zu einer kontrollierten Infektion, zum Vordringen in Organe und Gewebe des Organismus und zur Übertragung von DNA in abgelegene Körperzellen befähigen, und
- **daß** sie im Organismus ihrem typischen Infektionszyklus entsprechend und auf dem für sie typischen Infektionsweg das Zielorgan erreichen,
- **daß** der Infektionsweg natürlicherweise oder infolge gezielter genetischer Veränderungen gerichtet und örtlich begrenzt ist, wobei die gezielten genetischen Veränderungen insbesondere Gene betreffen, die die Vermehrung der Bakterien in den eukaryonten Zellen beeinflussen und/oder die Pathogenität der Bakterien im Organismus mindern und/oder das Überleben der Bakterien in der Umwelt nach einer Ausscheidung aus dem Organismus verhindern,
- und **daß** der Infektionszyklus durch den Einsatz von Antibiotika zeitlich begrenzbar und beendbar ist.

16. Verfahren nach Anspruch 15 unter Verwendung eines Bakteriums nach einem der Ansprüche 2 bis 13.

17. Verfahren nach Anspruch 15 oder 16 **dadurch gekennzeichnet,**
**daß** der Organismus ein Nutztier ist und daß die Transgenität im Euter von Milch produzierenden Tieren oder in Eiern von Geflügeltieren oder im Blut oder anderen Geweben des Nutztiers induziert wird.

18. Verwendung eines Bakteriums nach einem der Ansprüche 1 bis 15 zur Erzeugung eines Proteins für die Herstellung von Diagnostika oder Therapeutika, insbesondere Impfstoffen.

19. Verwendung nach Anspruch 18, **dadurch gekennzeichnet, dass** das Protein ein Hormon oder ein Regulationsfaktor oder ein Enzym oder ein Enzyminhibitor oder ein humaner monoklonaler Antikörper ist.

## Claims

1. Bacteria used as a vehicle for gene transport and gene transfer in eukaryotic cells of an organism for inducing a targeted somatic transgenesis in cells, tissue or organs, except the germ-line of the organism, whereby the bacteria comprise a foreign DNA integrated in an episomal vector, whose transcription and expression is controlled by eukaryotic regulator elements, **characterized in that**,
- the bacteria are vital and viable in the organism
- they are either (a) fully pathogenic,
or (b) at most attenuated in such way, that a prevention of the apoptosis induction of the eukaryotic cell, and/or a restriction of the intracellular motility of the bacteria, and/or an efficient elimination of the bacteria after the gene transfer is guaranteed,
or (c) naturally not pathogenic and provided with additional pathogenicity factors, which enable the bacteria to infect in a controlled manner, to advance into organs and tissue of the organism and to transfer DNA to remote somatic cells and
- they reach the target organ in the organism according to their typical cycle of infection and by their typical route of infection and are able to transmit the foreign DNA into remote somatic cells,
- the route of infection of the bacteria is directed and locally limited either naturally or due to specific genetic alterations, whereby the specific genetic alterations mainly concern genes, which influence the reproduction of the bacteria in the eukaryotic cells and/or reduce the pathogenicity of the bacteria in the organism and/or inhibit the survival of the bacteria in the environment after excretion from the organism,
- and the cycle of infection of the bacteria can be limited in time and terminated by using antibiotics.

2. Bacteria according to claim 1, **characterized in that**, transcription and expression of the foreign DNA is controlled by promoters and other regulatory sequences, which originate from the previously selected target organ of the TGC procedure or which are optimized for the target organ.

3. Bacteria according to claim 1 or 2, **characterized in that**, they are provided with an additional exogenous suicide gene.

4. Bacteria according to one of the claims 1 to 3, **characterized in that**, they are bacteria belonging to the genus Aeromonas, Bartonella, Brucella, Campylobacter, Clostridia, Enterobacteriaceae, Legionella, Listeria, Mycobacterium, Renibacterium, Rhodococcus or bacteria belonging to genera which are genetically or biochemically related to them.

5. Bacteria according to one of the claims 1 to 4, **characterized in that**, they contain a dapE gene with the nucleotide sequence shown in the sequence protocol SEQ ID NO. 1 or a gene matching at least in 35 % of the nucleotide positions, that gene being either deleted or inhibited by blocking or mutation.

6. Bacteria according to claim 5, **characterized in that**, they are bacteria belonging to the strain Listeria monocytogenes.

7. Bacteria according to one of the claims 1 to 5, **characterized in that**, they contain a cspL gene with the nucleotide sequence shown in the sequence protocol SEQ ID NO. 2 or a gene matching at least in 35 % of the nucleotide positions, that gene being either deleted or inhibited by blocking or mutation.

8. Bacteria according to claim 7, **characterized in that**, they are bacteria belonging to the genus Listeria.

9. Bacterial strain Listeria monocytogenes EGD HlyD_{491A}, which is deposited at the DSMZ under the number 11881 and is suitable for use according to claim 1.

10. Bacterial strain Listeria monocytogenes EGD Delta actA Delta plcB, which is deposited at the DSMZ under the number 11882 and is suitable for use according to claim 1.

11. Bacterial strain Listeria monocytogenes EGD Delta cspL 1, which is deposited at the DSMZ under the number 11883 and is suitable for use according to claim 1.

12. Bacteria according to one of the claims I to 11, **characterized in that**, they infect udders of cows or other lactating working animals.

13. Bacteria according to one of the claims 1 to 11, **characterized in that**, they infect the cells of the blood as somatic tissue.

14. The use of a bacterium according to one of the claims 1 to 13 for the production of proteins.

15. A method for the production and extraction of proteins in an animal organism except the human organism, where the bacteria comprising a foreign DNA integrated in an episomal vector, whose transcription and expression is controlled by eukaryotic regulator elements, are used as a vehicle for gene transport and gene transfer in eukaryotic cells tissue or organs, except the germ-line of the organism for inducing a targeted somatic transgenesis, and initiate the production of the foreign protein in the infected and transgenic cells, which is then isolated from the cell, tissue or organ and purified, **characterized in that**,
- the bacteria are vital and viable in the organism,
- the bacteria are either (a) fully pathogenic, or (b) at most attenuated in a way, that a prevention of the apoptosis induction of the eukaryotic cell, and/or a restriction of the intracellular motility of the bacteria, and/or an efficient elimination of the bacteria after the gene transfer is guaranteed, or (c) naturally not pathogenic and provided with additional pathogenicity factors, which enable the bacteria to infect in a controlled manner, to advance into organs and tissue of the organism and to transfer DNA to remote somatic cells, and
- they reach the target organ in the organism according to their typical cycle of infection and by their typical route of infection
- the route of infection of the bacteria is directed and locally limited either naturally or due to specific genetic alterations, whereby the specific genetic alterations mainly concern genes, which influence the reproduction of the bacteria in the eukaryotic cells and/or reduce the pathogenicity of the bacteria in the organism and/or inhibit the survival of the bacteria in the environment after excretion from the organism,
- and the cycle of infection of the bacteria can be limited in time and terminated by using antibiotics.

16. A method according to claim 15 using a bacterium according to one of the claims 2 to 13.

17. A method according to claim 15 or 16 **characterized in that**, the organism is a working animal and that the transgenesis is induced in the udder of lactating animals or in eggs of poultry or in the blood or other tissue of the working animal.

18. The use of a bacterium according to one of the claims 1 to 15 for the creation of a protein for the production of diagnostics or therapeutics, in particular vaccines.

19. The use according to claim 18, **characterized in that**, the protein is a hormone or a regulation factor or an enzyme or an enzyme inhibitor or a human monoclonal antibody.

## Revendications

1. Bactéries destinées à être utilisées comme véhicules pour le transport et le transfert génétique dans des cellules eucaryotes d'un organisme dans le but d'induire de façon ciblée une propriété transgénique somatique dans des cellules, des tissus ou des organes à l'exception de la lignée germinale de l'organisme, les bactéries présentant un ADN étranger intégré dans un vecteur épisomal, la transcription et l'expression de cet ADN étant sous le contrôle d'éléments eucaryotes régulateurs, **caractérisées en ce que**
- que les bactéries sont vitales et viables dans l'organisme,
- **en ce que** les bactéries sont soit (a) complètement pathogènes,
soit (b) tout au plus atténuées de telle sorte qu'un empêchement de l'induction de l'apoptose de la cellule eucaryotes et/ou qu'une limitation de la motilité intracellulaire des bactéries et/ou qu'une élimination efficace des bactéries après l'accomplissement du transfert génétique est assuré,
soit (c) les bactéries ne sont pas pathogènes de par leur nature et sont équipés de facteurs pathogènes supplémentaires qui rendent les bactéries capables d'une infection contrôlée , de pénétrer dans des organes et des tissus de l'organisme et de transmettre de l'ADN dans des cellules corporelles écartées, et
- en qu'elles avancent dans l'organisme vers l'organe et le tissu visés selon leur cycle d'infection typique et sur leur voie d'infection typique et qu'elles sont capables de transmettre de l'ADN étranger dans des cellules corporelles écartées,
- **en ce que** la voie d'infection est focalisée et limitée dans l'espace de façon naturelle ou suite à des modifications génétiques ciblées, les modifications génétiques ciblées concernant notamment les gènes qui influencent la multiplication des bactéries dans les cellules eucaryotes et/ou affaiblissent les capacités pathogènes des bactéries dans l'organisme et/ou empêchent la survie des bactéries dans l'environnement après une expulsion hors de l'organisme,
- et **en ce que** le cycle d'infection peut être limité dans le temps et être arrêté par l'utilisation d'antibiotiques.

2. Bactéries selon la revendication 1, **caractérisées en ce**
**que** la transcription et l'expression de l'ADN étranger se passe sous le contrôle de promoteurs et d'autres séquences à effet régulateur qui proviennent de l'organe ciblé préalablement par le procédé TGC ou qui sont optimisés pour l'organe ciblé.

3. Bactéries selon la revendication 1 ou 2, **caractérisées en ce**
**qu'**elles sont dotées d'un gène suicide exogène supplémentaire.

4. Bactéries selon l'une des revendications 1 à 3, **caractérisées en ce**
**que** ce sont des bactéries des souches d'Aeromonas, de Bartonella, de Brucella, de Campylobacter, de Clostridia, de Enterobacteriaceae, de Legionella, de Listeria, de Mycobacterium, de Renibacterium, de Rhodococcus ou des bactéries de souches apparentées biochimiquement ou génétiquement à celles-ci.

5. Bactéries selon l'une des revendications 1 à 4, **caractérisées en ce**
**qu'**elles possèdent un gène dapE avec la séquence de nucléotides illustrée dans le protocole de la séquence SEQ ID NO. 1 ou un gène qui y correspond dans au moins 35% des positions de nucléotides, lequel gène est délété ou inhibé par blocage ou par mutation.

6. Bactéries selon la revendication 5, **caractérisées en ce**
**que** se sont des bactéries de la souche de Listeria monocytogènes.

7. Bactéries selon l'une des revendications 1 à 5, **caractérisées en ce**
**qu'**elles possèdent un gène cspL avec la séquence de nucléotides illustrée dans le protocole de la séquence SEQ ID NO. 2 ou un gène qui y correspond dans au moins 35% des positions de nucléotides, lequel gène est délété ou inhibé par blocage ou par mutation.

8. Bactéries selon la revendication 7, **caractérisées en ce**
**que** se sont des bactéries de la souche de Listeria.

9. Souche de bactéries Listeria monocytogènes EGD HlyD_{491A}
qui est déposée à la DSMZ sous le numéro 11881 et qui est adaptée à l'utilisation selon la revendication 1.

10. Souche de bactéries Listeria monocytogènes EGD Delta actA Delta plcB
qui est déposée à la DSMZ sous le numéro 11882 et qui est adaptée à l'utilisation selon la revendication 1.

11. Souche de bactéries Listeria monocytogènes EGD Delta cspL 1
qui est déposée à la DSMZ sous le numéro 11883 et qui est adaptée à l'utilisation selon la revendication 1.

12. Bactéries selon l'une des revendications 1 à 11, **caractérisées en ce**
**qu'**elles infectent la mamelle de vaches ou d'autres animaux domestiques producteurs de lait.

13. Bactéries selon l'une des revendications 1 à 11, **caractérisées en ce**
**qu'**elles infectent des cellules du sang utilisées comme tissu somatique.

14. Utilisation d'une bactérie selon l'une des revendications 1 à 13 pour la production de protéines.

15. Procédé pour la production de protéines dans un organisme animal, à l'exception de l'organisme humain, dans lequel des bactéries qui possèdent un ADN étranger intégré dans un vecteur épisomal, dont la transcription et l'expression est sous le contrôle d'éléments eucaryotes régulateurs, sont utilisées comme véhicule pour le transport et le transfert génétique dans des cellules eucaryotes, des tissus ou des organes, à l'exception de la lignée germinale de l'organisme, dans le but d'induire de façon ciblée une propriété transgénique somatique, et qui initient dans des cellules infectées transgéniques la production d'une protéine étrangère qui, ensuite, est isolée de la cellule, du tissu ou de l'organe et purifiée, **caractérisé en ce**
- **que** les bactéries sont vitales et viables dans l'organisme,
- en ce que les bactéries sont soit (a) complètement pathogènes, soit (b) tout au plus atténuées de telle sorte qu'un empêchement de l'induction de l'apoptose de la cellule eucaryotes et/ou qu'une limitation de la motilité intracellulaire des bactéries et/ou qu'une élimination efficace des bactéries est assuré après la réalisation avec succès du transfert génétique, soit (c) ne sont pas pathogènes de par leur nature et sont équipées de facteurs pathogènes supplémentaires qui rendent les bactéries capables d'une infection contrôlée, de pénétrer dans des organes et des tissus de l'organisme et de transmettre de l'ADN dans des cellules corporelles écartées, et
- en ce qu'elles avancent dans l'organisme vers l'organe ciblé selon leur cycle d'infection typique et sur leur voie d'infection typique,
- en ce que la voie d'infection est focalisée et limitée dans l'espace de façon naturelle ou suite à des modifications génétiques ciblées, ces modifications génétiques ciblées concernant notamment les gènes qui influencent la multiplication des bactéries dans les cellules eucaryotes et/ou affaiblissent les capacités pathogènes des bactéries dans l'organisme et/ou empêchent la survie des bactéries dans l'environnement après une expulsion hors de l'organisme,
- et en ce que le cycle d'infection peut être limité dans le temps et être arrêté par l'utilisation d'antibiotiques.

16. Procédé selon la revendication 15 en utilisant une bactérie selon l'une des revendications 2 à 13.

17. Procédé selon la revendication 15 ou 16, **caractérisé en ce**
**que** l'organise est un animal domestique et en ce que la propriété transgénique est induite dans la mamelle d'animaux producteurs de lait ou dans les oeufs de volailles ou dans le sang ou autres tissus de l'animal domestique.

18. Utilisation d'une bactérie selon une des revendications 1 à 15 pour la
fabrication d'une protéine pour la production de substances pour le diagnostique ou de substances thérapeutiques, notamment de vaccins.

19. Utilisation selon la revendication 18, **caractérisée en ce que** la protéine est une hormone ou un facteur de régulation ou un enzyme ou un inhibiteur d'enzymes ou un anticorps humain monoclonal.
